(19) Europäisches Patentamt
European Patent Office
Office européen des brevets

(11) EP 0 648 120 B1

(12) **EUROPEAN PATENT SPECIFICATION**

(45) Date of publication and mention of the grant of the patent:
**29.12.1997 Bulletin 1997/52**

(21) Application number: **93914339.2**

(22) Date of filing: **04.06.1993**

(51) Int. Cl.$^6$: **A61K 31/675**, A61K 31/66

(86) International application number:
**PCT/US93/05267**

(87) International publication number:
**WO 94/00129 (06.01.1994 Gazette 1994/02)**

(54) **USE OF PHOSPHONATES FOR THE TREATMENT OF OSTEOPOROSIS**
VERWENDUNG VON PHOSPHINATE ZUR BEHANDLUNG DER OSTEOPOROSE
UTILISATION DE PHOSPHONATES DANS LE TRAITEMENT DE L'OSTEOPOROSE

(84) Designated Contracting States:
**AT BE CH DE DK ES FR GB GR IE IT LI LU NL PT SE**

(30) Priority: **30.06.1992 US 906609**

(43) Date of publication of application:
**19.04.1995 Bulletin 1995/16**

(73) Proprietor:
**PROCTER & GAMBLE PHARMACEUTICALS, INC.**
**Norwich, NY 13815 (US)**

(72) Inventors:
- **FRANCIS, Marion, David**
**Cincinnati, OH 45231 (US)**
- **BOYCE, Rogely, Waite**
**Pottstown, PA 19464 (US)**

(74) Representative:
**Brooks, Maxim Courtney et al**
**Procter & Gamble Technical Centres Limited,**
**Whitley Road,**
**Longbenton**
**Newcastle upon Tyne NE12 9TS (GB)**

(56) References cited:
**EP-A- 0 186 405**
**EP-A- 0 210 728**
**EP-A- 0 274 158**
**EP-A- 0 298 553**
**DE-A- 4 011 777**

- **DRUGS UNDER EXPERIMENTAL AND CLINICAL RESEARCH vol. XV, no. 9, 1989, pages 389 - 396 SIETSMA, W.K. ET AL 'ANTIRESORPTIVE DOSE-RESPONSE RELATIONSHIPS ACROSS THREE GENERATIONS OF BISPHOSPHONATES'**
- **THE NEW ENGLAND JOURNAL OF MEDICINE vol. 322, no. 18, 3 May 1990, pages 73 - 79 WATTS,N.B. ET AL 'INTERMITTENT CYCLICAL ETIDRONATE TREATMENT OF POSTMENOPAUSAL WOMEN' cited in the application**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 648 120 B1

## Description

BACKGROUND OF THE INVENTION

This invention relates to the use of a medicament for increasing bone mass in humans and other mammals, i.e., for the treatment of osteoporosis and related bone metabolic disorders. In particular, this invention relates to such a use by the administration of a low dose of a high potency phosphonate compound.

A number of pathological conditions which can afflict humans and other mammals involve abnormal calcium and phosphate metabolism. Such conditions may be divided into two broad categories:

(1) Conditions which are characterized by anomalous mobilization of calcium and phosphate leading to general or specific bone loss, such as osteoporosis and Paget's disease, or excessively high calcium and phosphate levels in the fluids of the body, such as hypercalcemia of tumor origin. Such conditions are sometimes referred to herein as pathological hard tissue demineralizations.

(2) Conditions which cause or result from deposition of calcium and phosphate anomalously in the body, such as arthritis, including rheumatoid arthritis and osteoarthritis. These conditions are sometimes referred to herein as pathological calcifications.

The first category includes the most common metabolic bone disorder, osteoporosis; osteoporosis is a condition in which bone hard tissue is lost disproportionately to the development of new hard tissue. Osteoporosis can be generally defined as the reduction in the quantity of bone, or the atrophy of skeletal tissue. Marrow and bone spaces become larger, fibrous binding decreases, and compact bone becomes fragile. Osteoporosis can be subclassified as menopausal, senile, drug-induced (e.g. adrenocorticoid, as can occur in steroid therapy); disease-induced (arthritic and tumor), etc.; however, the manifestations are essentially the same.

In general, there are two types of osteoporosis: primary and secondary. "Secondary osteoporosis" is the result of a separate disease process or agent. However, approximately 90% of all osteoporosis cases are "primary osteoporosis". Such primary osteoporosis includes postmenopausal osteoporosis, disuse osteoporosis age-associated osteoporosis (affecting a majority of individuals over the age of 70 to 80), and idiopathic osteoporosis affecting middle-aged and younger men and women.

For some osteoporotic individuals, the loss of bone tissue is sufficiently great so as to cause mechanical failure of the bone structure. Bone fractures often occur, for example, in the hip and spine of women suffering from postmenopausal osteoporosis. Kyphosis (abnormally increased curvature of the thoracic spine) may also result.

The mechanism of bone loss in osteoporotics is believed to involve an imbalance in the process of "bone remodeling". Bone remodeling occurs throughout life, renewing the skeleton and maintaining the strength of bone. This remodeling involves the erosion and filling of discrete sites on the surface of bones, by an organized group of cells called "basic multicellular units" or "BMUs". BMUs primarily consist of "osteoclasts", "osteoblasts", and their cellular precursors. In the remodeling cycle, bone is resorbed at the site of an "activated" BMU by an osteoclast, forming a resorption cavity. This cavity is then filled with bone by an osteoblast.

Normally, in adults, the remodeling cycle results in a small deficit in bone, due to incomplete filling of the resorption cavity. Thus, even in healthy adults, age-related bone loss occurs. However, in osteoporotics, there may be an increase in the number of BMUs that are activated. This increased activation accelerates bone remodeling, resulting in abnormally high bone loss.

Although its etiology is not fully understood, there are many risk factors thought to be associated with osteoporosis. These include low body weight, low calcium intake, physical inactivity, and estrogen deficiency.

Current osteoporosis treatment consists primarily of calcium and estrogen administration.

The second category, involving conditions manifested by anomalous calcium and phosphate deposition, includes myositis ossificans progressiva, calcinosis universal is, and such afflictions as arthritis (including, for example, rheumatoid arthritis and osteoarthritis), neuritis, bursitis, tendonitis, and other conditions which predispose involved tissue to deposition of calcium.

Many compositions and methods are described in the medical literature for the "treatment" of osteoporosis. Many of these compositions and methods attempt to either slow the loss of bone or to produce a net gain in bone mass. See, for example, R. C. Haynes, Jr. et al., "Agents affecting Calcification", The Pharmacological Basis of Therapeutics, 7th Edition (A. G. Gilman, L. S. Goodman et al., Editors, 1985); G. D. Whedon et al., "An Analysis of Current Concepts and Research Interest in Osteoporosis", Current Advances in Skeletogenesis (A. Ornoy et al., Editors, 1985); and W. A. Peck, et al., Physician's Resource Manual on Osteoporosis (1987), published by the National Osteoporosis Foundation.

Among the treatments for osteoporosis suggested in the literature is the administration of bisphosphonates or other bone-active phosphonates. See, for example, Storm et al., "Effect of Intermittent Cyclical Etidronate Therapy on Bone Mineralization and Fracture Rate in Women with Post-Menopausal Osteoporosis", 322 New England Journal of Medicine 1265 (1990); and Watts et al., "Intermittent Cyclical Etidronate Treatment of Post-Menopausal Osteoporosis", 323 New England Journal of Medicine 73 (1990). Such treatments using a variety of bisphosphonates are described in U.S. Patent 4,761,406, Flora et al., issued August 2, 1988; U.S. Patent 4,812,304, Anderson et al., issued March 14, 1989; U.S. Patent 4,812,311, Uchtman, issued March 14, 1989; and U.S. Patent 4,822,609, Flora, issued April 18, 1989. The use of such phosphonates for the treatment of osteoporosis, and other disorders involving abnormal calcium and phosphate metabolism, is also described in U.S. Patent 3,683,080, Francis, issued August 8, 1972; U.S. Patent 4,330,537, Francis, issued October 28, 1980; U.S. Patent 4,267,108, Blum et al., issued May 12, 1981; European Patent Publication 298,553, Ebetino, published January 11, 1989; and Francis et al., "Chemical, Biochemical, and Medicinal Properties of the Diphosphonates", The Role of Phosphonates in Living Systems, Chapter 4 (1983).

An osteoporotic patient may be on bisphosphonate therapy for as long as two years on a daily basis. Such therapy is not without side effects, however. Ethane-1-hydroxy-1,1-Diphosphonate (EHDP) at levels of 5 mg/kg (1.25 mgP/kg) s.c. in rats and dogs, for 180 and 140 consecutive days, respectively, causes a decrease in bend and break strength in rat femurs and an increase in cortical porosity indicating active resorption in dogs. See Lenehan, T., et al., "Effect of EHDP on Fracture Healing in Dogs", 3(4) J. Orthop. Res. 499-507 (1985) and Shiota, E. "Effects of Diphosphonate on Osteoporosis Induced in Rats", 76(6) Fukuoka Acta Med. 317-342 (1985).

In order to alleviate the side effects of bisphosphonate therapy, investigators have experimented with continuous, long term, low doses of bisphosphonates, specifically, the low potency bisphosphonate, ethane-1-hydroxy-1,1-diphosphonate. See Shiota, E., et al., "Ethane-1-Hydroxy-1,1-Diphosphonate (EHDP) The Effects of Long-Term, Low Dose EHDP on Experimental Osteoporosis", 31 Seikei Geka to Saigai Geka 681-683 (1983), Shiota, E., et al., "EHDP-Effects of Long Term, Low-Dose EHDP on Experimental Osteoporosis Induced by Sciatic Nerve Dissection in Rats", 32 Seikei Geka to Saigai Geka 772-776 (1984), and Shiota, Et., et al., "The Effects of Long-Term, Low Dose EHDP on Experimental Osteoporosis Induced by Ovariectomy and Low Calcium Diet in Rats", 33 Seikei Geka to Saigai Geka 196-199 (1984), and Lenehan, T., et al., "Effect of EHDP on Fracture Healing in Dogs", 3(4) J. Orthop. Res. 499-507 (1985).

Continuous, long term, low dosage regimens comprising administering 0.1 mg/kg (0.025 mgP/kg), 0.5 mg/kg (0.125 mgP/kg), 1.0 mg/kg (0.25 mg P/kg) and 2.0 mg/kg (0.50 mgP/kg) s.c. doses of EHDP, a low potency bisphosphonate compound, to rats and dogs, for 180 and 140 day periods, respectively, resulted in an increase in bone mineral content, cortical thickness, bone ash, and break and bend strength.

Similarly, continuous, long term, low dose administration of the medium potency bisphosphonate, 3-amino-1-hydroxypropylidene-1,1-bisphosphonate (APD), at a dosage range of 0.0045 mg/kg (0.001 mgP/kg) - 0.45 mg/kg (0.1 mgP/kg), resulted in an increase in bone quality and mass while APD at levels of 1.4 mg/kg (0.3 mgP/kg) and above resulted in a decrease in bone quality and mass. *See* Ferretti, J.L., et al., "Biomechanical Effects of the full range of useful doses of 3-amino-1-hydroxypropylidene-1,1-bisphonate (APD) in femur diaphyses and cortical bone tissue in rats", 11(1) Bone Mineral 111-122 (1990).

Clearly, those skilled in the art have disclosed that the therapeutic efficacy of the continuous, long term administration of low and medium potency bisphosphonates is maintained at low doses. Furthermore, untoward side effects are reduced. However, the art has not disclosed the long term, low level administration of high potency phosphonate compounds, which include bisphosphonates, phosphonoalkylphosphinates, phosphonosulfonates and phosphonocarboxylates.

Applicant has found that high potency phosphonate compounds can be administered long term at low-dose levels on both a continuous and noncontinuous basis. Accordingly, the use of of this invention provides effective methods of preventing and treating osteoporosis, with reduced side effects compared to such methods known in the art.

## SUMMARY OF THE INVENTION

A use for increasing bone mass in a human or other mammal subject afflicted with osteoporosis, comprising a thirty (30) day treatment period, comprised of a high potency phosphonate compound administration regimen wherein

(a) said high potency phosphonate administration regimen comprises the systemic administration to said subject of a high potency phosphonate compound at a level of from 0.00001 mgP/kg to 0.1 mgP/kg per day that said high potency phosphonate compound is administered, provided that said level of the high potency phosphonate compound is administered at least 1 day of every said thirty (30) day treatment period; and wherein
(b) said thirty (30) day treatment period may be followed by a rest period of at least one day.

### Definitions and Usage of Terms

The following is a list of definitions for terms used herein.

"Heteroatom" is a nitrogen, sulfur, or oxygen atom. Groups containing one or more heteroatoms may contain different heteroatoms.

"Alkyl" is an unsubstituted or substituted, straight-chain or branched, saturated or unsaturated hydrocarbon chain, said hydrocarbon chain may be saturated, having 1 to 8 carbon atoms, and preferably, unless otherwise stated, from 1 to 4 carbon atoms; said hydrocarbon chain may be unsaturated, having 2 to 8 carbon atoms, and preferably, unless otherwise stated, 2 to 4 carbon atoms. Accordingly, the term "alkyl", as used herein, encompasses alkenyl hydrocarbon unsaturated chains having at least one olefinic double bond and alkynyl hydrocarbon unsaturated chains having at least one triple bond. Preferred alkyl groups include, but are not limited to, methyl, ethyl, propyl, isopropyl, and butyl.

"Heteroalkyl" is an unsubstituted or substituted, saturated chain having from 3 to 8-members and comprising carbon atoms and one or two heteroatoms.

"Carbocyclic ring" or "Carbocycle" as used herein is an unsubstituted or substituted, saturated, unsaturated or aromatic, hydrocarbon ring, generally containing from 3 to 8 atoms, preferably from 5 to 7, atoms. Carbocyclic rings may be monocyclic, having from 3 to 8, preferably from 5 to 7, carbon atoms, or they may be polycyclic. Polycyclic carbocycles consisting of two rings generally have from 6 to 16, preferably from 10 to 12, atoms. Polycyclic carbocycles consisting of three rings generally contain from 13 to 17, preferably from 14 to 15, atoms.

"Heterocyclic ring" or "heterocycle" as used herein is an unsubstituted or substituted, saturated, unsaturated or aromatic ring comprised of 3 to 8, preferably 5-7 carbon atoms, and one or more additional heteroatoms in the ring. The term "heterocyclic ring moieties" as used herein encompasses monocyclic or polycyclic ring systems, fused or unfused, unsaturated, saturated or unsubstituted. Monocyclic heterocyclic ring moieties generally contain from 3 to 8 atoms, preferably from 5 to 7, atoms. Polycyclic heterocyclic ring moieties consisting of two rings generally contain from 6 to 16, preferably from 10 to 12, atoms. Polycyclic heterocyclic ring moieties consisting of three rings generally contain from 13 to 17 atoms, preferably from 14 to 15, atoms. In addition, a polycyclic heterocyclic ring moiety may consist solely of heterocycles (one of which must contain a nitrogen atom), or of both heterocycles (one of which must contain a nitrogen atom) and carbocycles. Each heterocyclic ring moiety must have at least one nitrogen atom. Unless otherwise stated, any additional heteroatom in the heterocyclic ring moiety may be independently chosen from nitrogen, sulfur, and oxygen.

"Aryl" is an aromatic carbocyclic ring. Preferred aryl groups include, but are not limited to, phenyl, tolyl, xylyl, cumenyl, and naphthyl

"Heteroaryl" is an aromatic heterocyclic ring. Preferred heteroaryl groups include, but are not limited to, thienyl, furyl, pyrrolyl, pyridinyl, pyrazinyl, oxazolyl, thiazolyl, quinolinyl, pyrimidinyl, and tetrazolyl.

"Alkoxy" is an oxygen atom having a hydrocarbon chain substituent, where the hydrocarbon chain is an alkyl or alkenyl (e.g., -O-alkyl or -O-alkenyl). Preferred alkoxy groups include, but are not limited to, methoxy, ethoxy, propoxy, and alkyloxy.

"Hydroxyalkyl" is a substituted hydrocarbon chain which has a hydroxy substituent (e.g., -OH), and may have other substituents. Preferred hydroxyalkyl groups include, but are not limited to, hydroxyethyl, hydroxypropyl, and hydroxyalkyl.

"Carboxyalkyl" is a substituted hydrocarbon chain which has a carboxy substituent (e.g. -COOH) and may have other substituents. Preferred carboxyalkyl groups include carboxymethyl, carboxyethyl, and their acids and esters.

"Aminoalkyl" is a hydrocarbon chain (e.g. alkyl) substituted with an amine moiety (e.g., alkyl-NH-) such as methyl amine.

"Alkylamino" is an amino moiety having one or two alkyl substituents (e.g., -N-alkyl), such as dimethylamine.

"Alkenylamino" is an amino moiety having one or two alkenyl substituents (e.g., -N-alkenyl).

"Alkynalamino" is an amino moiety having one or two alkynyl substituents (e.g., -N-alkynyl).

"Alkylimino" is an imino moiety having one or two alkyl substituents (e.g., -N-alkyl-).

"Arylalkyl" is an alkyl moiety substituted with an aryl group. Preferred arylalkyl groups include benzyl and phenylethyl.

"Arylamino" is an amine moiety substituted with an aryl group (e.g., -NH-aryl).

"Aryloxy" is an oxygen atom having an aryl substituent (e.g., -O-aryl).

"Acyl" or "carbonyl" is a carbon to oxygen double bond, (e.g., R-C(=O)-). Preferred alkylacyl groups include, but are not limited to, acetyl, propionyl, butanoyl and benzoyl.

"Acyloxy" is an oxygen atom having an acyl substituent (e.g., -O-acyl); for example, -O-C(=O)-alkyl.

"Acylamino" is an amino moiety having an acyl substituent (e.g., -N-acyl); for example, -NH-(C=O)-alkyl.

"Halo", "halogen", or "halide" is a chloro, bromo, fluoro, or iodo atom radical. Chloro, bromo, and fluoro are preferred halides.

Also, as referred to herein, a "lower" hydrocarbon moiety (e.g., "lower" alkyl) is a hydrocarbon chain comprised of from, unless otherwise stated, 1 to 6, preferably from 1 to 4, carbon atoms.

As used herein, the term "thio-substituent" is depicted by $SR^6$ or $R^8SR^6$, wherein $R^8$ is a $C_1$-$C_8$ alkyl. Particular thio-substituents include thiol (-SH, where $R^6$ = H); thioesters

$$(S\text{-}\overset{O}{\overset{\|}{C}}R^7,$$

where $R^6$ is $COR^7$); thiocarbamates

$$(S\text{-}\overset{O}{\overset{\|}{C}}\text{-}NR^7,$$

where $R^6$ is $CONR^7$); dithiocarbamates

$$(S\text{-}\overset{S}{\overset{\|}{C}}\text{-}NR^7,$$

where $R^6$ is $CSNR^7_2$); dithioesters

$$(\text{-}S\text{-}\overset{S}{\overset{\|}{C}}R^7,$$

where $R^6$ is $CSR^7$); thiocarbonates

$$(S\text{-}\overset{O}{\overset{\|}{C}}\text{-}OR^7,$$

where $R^6$ is $C(O)OR^7$), and dithiocarbonates

$$(S\text{-}\overset{S}{\overset{\|}{C}}\text{-}OR^7,$$

where $R^6$ is $C(S)OR^7$). $R^7$ as used herein is hydrogen or substituted or unsubstituted $C_1$-$C_8$ alkyl. It is to be understood that the $SR^6$ groups defined above can be preceded by an $R^8$ (i.e. a $C_1$-$C_8$ alkyl); this would yield alkyl thiols, alkyl thioesters, alkyl dithioesters, alkyl thiocarbamates, alkyl dithiocarbamates, alkyl thiocarbonates and alkyl dithiocarbonates.

The terms "bisphosphonate" or "bisphosphonic acid" as used herein relate to those phosphonate or phosphonic acids that have two phosphonate groups attached to the same carbon atom and are used interchangeably with the terms diphosphonate and diphosphonic acids. Using the structures described herein, in these compounds the moiety R is $PO_3H_2$.

A "pharmaceutically-acceptable" salt is the salt formed from the interaction between any acid (e.g. phosphoric; carboxylic; and phosphonic) and any base (NaOH; KOH; and $NH_4OH$) and between an acid (HCl; $H_2SO_4$; and $HNO_3$) and any basic group (-OH; primary, tertiary, and secondary amines). Many such salts are known in the art, as described in World Patent Publication 87/05297, Johnston et al., published September 11, 1987, incorporated by reference herein.

A "pharmaceutically-acceptable ester" is the ester formed from the interaction between any acid and any alcohol or other organic compound rich in hydroxy groups. For example, esters of acetic acid are called acetates and esters of phosphonic acids are called phosphonates. Many such esters are known in the art, as described in World Patent Publication 87/05297, Johnston et al., published September 11, 1987, and hereby incorporated by reference herein. Such esters include lower alkyl esters, lower acyloxyalkyl esters (such as acetoxymethyl, acetoxyethyl, aminocarbonyloxymethyl, pivaloyloxymethyl, and pivaloyloxyethyl esters), lactonyl esters (such as phthalidyl and thiophthalidyl esters), lower alkoxyacyloxyalkyl esters (such as methoxycarbonyloxymethyl, ethoxycarbonyloxyethyl and isopropoxycarbonyloxyethyl esters), alkoxyalkyl esters, choline esters, and acylamino alkyl esters (such as acetamidomethyl esters).

As defined above and as used herein, substituent groups may themselves be substituted. Such substitution may be with one or more substituents. Such substituents include, but are not limited to, those listed in C. Hansch and A. Leo, Substituent Constants for Correlation Analysis in Chemistry and Biology (1979), hereby incorporated by reference herein. Preferred substituents include, but are not limited to, alkyl, alkenyl, alkoxy, hydroxy, oxo, amino, aminoalkyl (e.g. aminomethyl, etc.), cyano, halo, carboxy, alkoxyacetyl (e.g. carboethoxy, etc.), thio, thiol, aryl, cycloalkyl, heteroaryl, heterocycloalkyl (e.g., piperidinyl, morpholinyl, piperazinyl, pyrrolidinyl, etc.), imino, thioxo, hydroxyalkyl, aryloxy, arylalkyl, and combinations thereof.

DESCRIPTION OF THE INVENTION

The subject-matter of the present invention comprises the use of low levels of a high potency phosphonate compound in the treatment of a human or other mammal subject. Specific compounds and compositions to be used in these processes must, accordingly, be pharmaceutically-acceptable. As used herein, such a "pharmaceutically-acceptable" component is one that is suitable for use with humans and/or animals without undue adverse side effects (such as toxicity, irritation, and allergic response) commensurate with a reasonable benefit/risk ratio. Further, as used herein, the term "safe and effective amount" refers to the quantity of a component which is sufficient to yield a desired therapeutic response without undue adverse side effects (such as toxicity, irritation, or allergic response) commensurate with a reasonable benefit/risk ratio when used in the manner of this invention. The specific "safe and effective amount" will, obviously, vary with such factors as the particular condition being treated, the physical condition of the patient, the duration of the treatment, the nature of concurrent therapy (if any), and the specific formulations employed.

Active Materials

Phosphonate Compounds:

The use of this invention involves the administration of low levels of a high potency phosphonate compound. The term "high potency", as used herein, denotes those phosphonates having a subcutaneous LED of 0.01 mgP/kg to 0.0001 mgP/kg. The potency of a particular phosphonate can be expressed in terms of its "LED" or "least effective dose", which is the minimum dose of phosphonate expressed in mg P/kg that is effective, by itself, to cause a significant inhibition of bone resorption, i.e. the anti-resorptive dose. The specific LEDs of the phosphonates will vary depending upon their chemical composition, and their method of administration (i.e., oral or parenteral). The lower the LED, the more potent the anti-resorptive effect of the phosphonate.

As referred to herein, a "phosphonate compound" includes one or more compounds of the general formula:

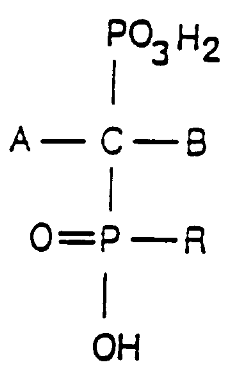

(1)

and pharmaceutically-acceptable salts and esters thereof, wherein A, B, and R are as defined hereinafter.

In Formula (1), "R" is hydroxy (for bisphosphonates), hydrogen or $C_1$-$C_8$ alkyl (for phosphonoalkylphosphinates), carboxylic acid (for phosphonocarboxylates), or sulfonic acid (for phosphonosulfonates). In the phosphonoalkylphosphinates, R is preferably unsubstituted alkyl, especially $C_1$-$C_8$ alkyl. When R is substituted $C_1$-$C_8$ alkyl, preferred substituents include halogen, unsubstituted or substituted phenyl, unsubstituted or substituted pyridinyl, unsubstituted amino, amino substituted with one or two lower alkyl groups, hydroxy, or carboxy. More preferred substituents are fluoro, phenyl, unsubstituted amino, and hydroxy; most preferred are fluoro (especially when present as trifluoromethyl) and phenyl.

Particularly preferred R moieties in the phosphonoalkylphosphinates are unsubstituted $C_1$-$C_8$ alkyl groups, especially unsubstituted, straight-chain, saturated lower alkyl groups. Also preferred R moieties are methyl, ethyl, n-propyl, iso-propyl, n-butyl, iso-butyl, sec-butyl, tert-butyl, and n-hexyl. More preferably, R is methyl, ethyl, n-propyl, or n-butyl. Most preferably, R is methyl.

In Formula (1), "A" is nitro; substituted or unsubstituted alkyl; substituted or unsubstituted heterocycle; thio-substituted heterocycle; substituted or unsubstituted quaternary nitrogen containing heterocycle; thio-substituted quaternary nitrogen containing heterocycle; aryl; heteroaryl; thio-substituted heteroaryl; substituted or unsubstituted quaternary nitrogen containing heteroaryl; thio-substituted quaternary nitrogen containing heteroaryl; unsubstituted amino, or the carboxylic acid amide thereof; amino substituted with one substituent group, or the carboxylic acid amide thereof; amino substituted independently with one alkyl group, or the carboxylic acid ester thereof; ether having a substituent group; thiol, thioester, dithioester, thiocarbamate, dithiocarbamate, thiocarbonate, dithiocarbonate, alkylthiol, alkylthioester, alkyldithioester, alkylthiocarbamate, alkyldithiocarbamate, alkylthiocarbonate, and alkyldithiocarbonate; thioether having a substituent group, or the sulfoxide and sulfone derivative thereof; $-SO_3H$, the pharmaceutically-acceptable salts thereof, the alkyl ester thereof; the unsubstituted amide thereof, or the amide thereof substituted with one or two alkyl groups; $-CO_2H$, the pharmaceutically-acceptable salts thereof, the alcohol ester thereof, the unsubstituted amide thereof, or the amide thereof substituted with one or two alkyl groups; aldehyde; ketone having a substituent group; carbamate, unsubstituted or substituted with one or two alkyl groups; peptides having from about 1 to about 100 amino acid moieties; or the A and B moieties are covalently linked to form a ring having from 3 to 7 atoms with from 0 to 3 heteroatoms selected from the group consisting of nitrogen, sulfur, phosphorus and oxygen, the ring being unsubstituted or substituted with one or more of the above substituents of A; or the A and B moieties are replaced by an unsubstituted or substituted alkyl moiety attached to the geminal carbon (the carbon shown in structure (1) hereinabove) by a double bond.

Preferably, A is one of the following moieties.

(1) substituted or unsubstituted alkyl having the general structure:

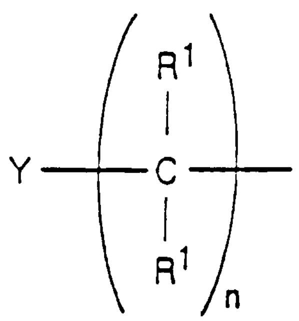

(2)

wherein:

(a) n is an integer from 1 to 10, preferably from 1 to 5, more preferably 1 or 2, more preferably 1;

(b) each $R^1$ is, independently, hydrogen, halogen, $C_1$-$C_8$ alkyl, $R^{10}SR^6$, $SR^6$ (where $R^6$ is H,-C(O)$R^7$, C(O)O$R^7$, C(S)O$R^7$, C(S)$R^7$, C(O)N$R^7{}_2$, C(S)N$R^7{}_2$, and $R^7$ is H or $C_1$-$C_8$ alkyl; and $R^{10}$ is substituted or unsubstituted $C_1$-$C_8$ alkyl), unsubstituted amino or the carboxylic acid amide thereof; amino substituted with one lower alkyl group or the carboxylic acid amide thereof; amino substituted independently with two lower alkyl groups, hydroxy or the carboxylic acid ester thereof; $-CO_2H$ or the pharmaceutically-acceptable salts thereof or the lower alcohol ester thereof; the unsubstituted amide thereof or the amide thereof substituted with one or two lower alkyl groups, ether having a lower alkyl group, $-PO_3H_2$ or the pharmaceutically-acceptable salts thereof, and nitro, or two $R^1$'s on the same carbon atom are =O or =N$R^9$ (where $R^9$ is lower alkyl or may be hydrogen when there is another nitrogen atom attached to the same carbon atom as the =N$R^9$ moiety), or two $R^1$'s on adjacent carbon atoms may be replaced by an additional bond between the carbon atoms; or an $R^1$ on the first carbon atom (from the right side of structure (2) hereinabove) and B (see structure (1) hereinabove) may be replaced by an additional bond; and

(c) Y is halogen; nitro; cyano; substituted or unsubstituted heterocycle; thio-substituted heterocycle; subsituted or unsubstituted quaternary nitrogen containing heterocycle; thio-substituted quaternary nitrogen containing heterocycle; aryl; substituted or unsubstituted heteroaryl; thio-substituted heteroaryl; substituted or unsubstituted quaternary nitrogen containing heteroaryl; thio-substituted quaternary nitrogen containing heteroaryl; unsubstituted amino, and the carboxylic acid amide thereof; amino substituted with one alkyl, heterocycle, aryl or heteroaryl group and the carboxylic acid amide thereof; amino substituted independently with one alkyl group and one heterocycle, aryl or heteroaryl group; hydroxy, and the carboxylic ester thereof; thiol, and the carboxylic acid thiol ester thereof; thioether having an alkyl, heterocycle, aryl or heteroaryl group, and the sulfoxide and sulfone derivatives thereof; $-SO_3H$, the pharmaceutically-acceptable salts thereof, the lower alkyl ester thereof; the unsubstituted amide thereof, and the amide thereof substituted with one or two alkyl groups; $-CO_2H$, the pharmaceutically-acceptable salts thereof, the lower alcohol ester thereof; $PO_3H_2$, the pharmaceutically-acceptable salts thereof, the lower alcohol ester thereof; the unsubstituted amide thereof, and the amide thereof substituted with one or two alkyl groups; $-(R^8)PO_2H$ (where $R^8$ is hydrogen or unsubstituted lower alkyl), the pharmaceutically-acceptable salts thereof, the alcohol ester thereof; the unsubstituted amide thereof, and the amide thereof substituted with one or two alkyl groups; aldehyde; ketone having an alkyl group; carbamate, unsubstituted or substituted with one or two alkyl groups; or peptidyl. Y is preferably a substituted or unsubstituted heterocycle (preferably 5 to 7 membered heterocycles having one or two nitrogen atoms); a substituted or unsubstituted quaternary nitrogen containing heterocycle; amino; and substituted amino. Particularly preferred Y moieties include pyridyl, pyridinium, piperidinyl, piperidinium, amino, and amino substituted with one or two lower alkyl groups. Preferably, for phosphonoalkylphosphinates, Y is halogen (preferably fluoro); trifluoromethyl; ether having a lower alkyl group; unsubstituted amino, and the amide thereof derived from a carboxylic acid of a lower alkyl group, amino substituted with one lower alkyl group and the amide thereof derived from carboxylic acid of a lower alkyl group; amino substituted independently with two lower alkyl groups; or peptidyl having from one to about six amino acid moieties.

(2) substituted or unsubstituted cycloalkyl having from 4 to 10 carbon atoms, preferably 5 or 6 carbon atoms
(3) thio-substituted cycloalkyl having from 4 to 10 carbon atoms, preferably 5 or 6 carbon atoms
(4) substituted or unsubstituted heterocycle having 5 to 12 atoms in the ring; more preferably one or two nitrogen atoms in the ring, more preferably having one nitrogen atom in the ring. Particularly preferred heterocycles are unsubstituted or substituted piperidinyl, pyrrolidinyl, piperazinyl, and morpholinyl
(5) thio-substituted heterocycle having 5 to 12 atoms in the ring; more preferably one or two nitrogen atoms in the ring, more preferably having one nitrogen atom in the ring. Particularly preferred thiol-substituted heterocycles are unsubstituted or substituted [(5-[mercaptomethyl]-2-piperidinyl)methylene]bis-[phosphonic acid]; [2-(3-mercaptomethyl-5-methyl-2-pyridinyl)-ethylidene-bis[phosphonic acid]; [(5-mercapto-4-piperidinyl)methylene]-bis[phosphonic acid].
(6) substituted or unsubstituted quaternary nitrogen containing heterocycle having 5 or 12 atoms in the ring. Particularly preferred quaternary nitrogen containing heterocycles are unsubstituted or substituted piperidinium, pyrrolidinium, and piperazinium
(7) thio-substituted quaternary nitrogen containing heterocycle having 5 or 12 atoms in the ring. Particularly preferred thiol-substituted quaternary nitrogen containing heterocycles are unsubstituted or substituted 4-[(diphosphonomethyl)thio]-1,1-dimethylpiperidinium chloride; 3-(2,2-diphosphonoethyl)-1-(2-mercaptoethyl)pyridinium chloride.
(8) unsubstituted and substituted phenyl and naphthyl
(9) unsubstituted and substituted 5- to 12-membered ring heteroaryls having one or two heteroatoms (especially nitrogen heteroatoms), preferably pyridinyl
(10) unsubstituted and substituted 5- to 12-membered ring quaternary nitrogen containing heteroaryls, preferably, pyridinium.
(11) an amine-containing moiety having the general structure:

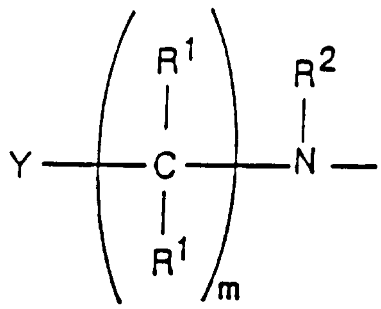

(3)

wherein

(a) m is an integer from 0 to 10, preferably from 0 to 5, more preferably 0 or 1, more preferably 0;
(b) $R^1$ and Y are as described hereinbefore; and
(c) $R^2$ is hydrogen, lower alkyl or acyl derived from a carboxylic acid of a lower alkyl

(12) an amine-containing moiety having the general structure:

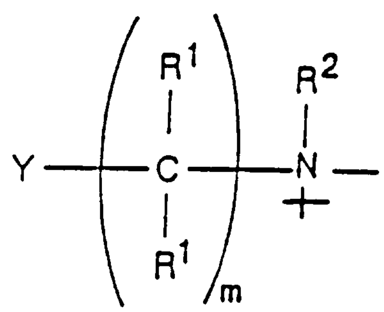

wherein

(a) the nitrogen atom is quaternized
(b) m is an integer from 0 to 10, preferably from 0 to 5, more preferably 0 or 1, more preferably 0;
(c) $R^1$ and Y are as described hereinbefore; and
(d) $R^2$ is hydrogen, lower alkyl or acyl derived from a carboxylic acid of a lower alkyl

(13) an oxygen-containing moiety having the general structure:

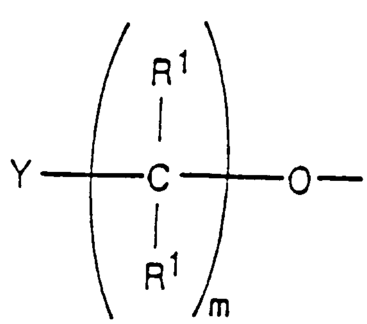

(4)

wherein

(a) m is an integer from 0 to 10, preferably from 0 to 5, more preferably 0 or 1, more preferably 0; and
(b) $R^1$ and Y are as described hereinbefore

(14) sulfur-containing moiety having the general structure:

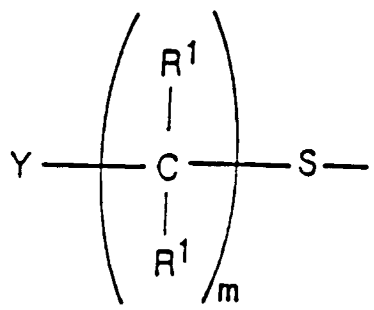

(5)

wherein

(a) m is an integer from 0 to 10, preferably from 0 to 5, more preferably 0 or 1, more preferably 0; and
(b) $R^1$ and Y are as described hereinbefore

In Formula (1), "B" is hydrogen; halogen; unsubstituted or substituted lower alkyl; unsubstituted or substituted cycloalkyl having from 4 to 10 atoms in the ring; thio-substituted cycloalkyl; unsubstituted or substituted heterocycle having from 5 to 12 atoms in the ring; thio-substituted heterocycle having 5 to 12 atoms in the ring; unsubstituted or substituted quaternary nitrogen containing heterocycle having 5 to 12 atoms in the ring; thio-substituted quaternary nitrogen containing heterocycle having 5 to 12 atoms in the ring; unsubstituted or substituted phenyl; hydroxy, or the carboxylic acid ester thereof; thiol; unsubstituted amino, or the carboxylic acid amide thereof; amino substituted with one lower alkyl group, or the carboxylic acid amide thereof; amino substituted independently with two lower alkyl groups; or $-CO_2H$, the pharmaceutically-acceptable salts thereof, the alcohol ester thereof; the unsubstituted amide thereof, or the amide thereof substituted with one or two lower alkyl groups.

To maintain chemical stability of these compounds, the A and B moieties preferably do not both have heteroatoms (nitrogen, oxygen or sulfur), or a heteroatom and a halogen, bonded to the phosphonate moiety (i.e., the carbon atom geminally substituted with the phosphorous atoms). Thus, when the A moiety has an oxygen, sulfur, nitrogen, or halogen atom bonded to the phosphorous-substituted methylene carbon, then B is selected from hydrogen; unsubstituted or substituted lower alkyl, cycloalkyl, heterocycle (where a carbon atom of the heterocycle is bonded to the geminal carbon atoms), or phenyl ; $-CO_2H$, the pharmaceutically-acceptable salts thereof, the ester thereof derived from an alcohol of a lower alkyl group, the unsubstituted amide thereof, and the amide thereof substituted with one or two lower alkyl groups.

Preferably B is hydrogen, halogen, unsubstituted or substituted lower alkyl, unsubstituted or substituted cycloalkyl having 4 to 10 carbons, thio-substituted cycloalkyl having 4 to 10 carbons; unsubstituted or substituted phenyl, unsubstituted or substituted benzyl, hydroxy or the carboxylic acid ester thereof; thiol, unsubstituted amino or the carboxylic acid amide thereof; amino substituted with one lower alkyl group or the carboxylic acid amide thereof; amino substituted independently with two lower alkyl groups, or $-CO_2H$ or the pharmaceutically-acceptable salts thereof and the lower alcohol ester thereof; and the unsubstituted amide thereof or the amide thereof substituted with one or two lower alkyl groups.

More preferably, B is hydrogen, chloro, methyl, ethyl, unsubstituted or substituted cycloalkyl having 4 to 10 carbons, hydroxy, thiol, unsubstituted amino, (N-methyl)amino, (N,N-dimethyl)amino, $-CO_2H$ or the pharmaceutically-acceptable salts thereof, $-CO_2CH_3$, or $-CONH_2$. More preferably, B is hydrogen, methyl, chloro, amino, hydroxy, or thiol.

When A is a heterocycle having 5 to 12 carbons and B is a cycloalkyl having 4 to 10 carbons, A and B can be fused to form a polycyclic ring. Said polycyclic ring may contain a quaternary nitrogen heteroatom. Said polycyclic ring may be thio-substituted. Preferred polycyclic ring phosphonates are octahydro-1-mercapto-2-pyrindine-6,6-bisphosphonic acid; octahydro-1,1-dimethyl-5,5-diphosphono-1-pyrindinium salt; and (7-dihydro-1-pyrindine)methane bisphosphonic acid.

Particularly preferred phosphonate compounds useful herein are of the formula:

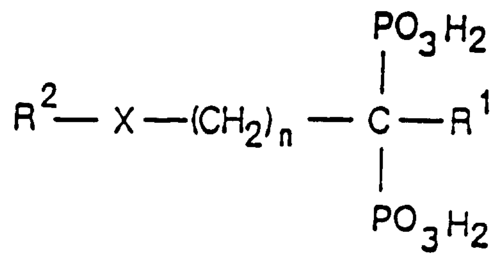

(6)

wherein: n is an integer from 0 to 7 (preferably from 0 to 2, more preferably 1); $R^1$ is hydrogen, $R^{10}SR^6$, $SR^6$ (where $R^6$ is H, $-C(O)R^7$, $C(O)OR^7$, $C(S)OR^7$, $C(S)R^7$, $C(O)NR^7_2$, $C(S)NR^7_2$, and $R^7$ is H or $C_1$-$C_8$ alkyl; $R^{10}$ is substituted or unsubstituted $C_1$-$C_8$ alkyl), chloro, amino, or hydroxy (preferably hydrogen or hydroxy); X is -NH-, oxygen, sulfur, or a single bond (preferably -NH- or single bond); $R^2$ is a substituted or unsubstituted 5- to 12-membered heterocycle having from 1 to 3 heteroatoms (preferably a 6-membered heterocycle having 1 or 2 nitrogen atoms), a substituted or unsubstituted 4- to 12-membered polycyclic ring, a substituted or unsubstituted 5- to 12-membered quaternary nitrogen containing heterocycle, a substituted or unsubstituted 4- to 12-membered quaternary nitrogen containing polycyclic ring, a thio-substituted 5- to 12-membered heterocycle, a thio-substituted 4- to 12-membered polycyclic ring, a thio-substituted 5- to 12-membered quaternary nitrogen containing heterocycle, a thio-substituted 4- to 12-membered quaternary nitrogen containing polycyclic ring, amino, amino substituted with one or two lower alkyl groups, or hydrogen; and their pharmaceutically-acceptable salts and esters.

The term "pharmaceutically-acceptable salts and esters", as used herein, means hydrolyzable esters and salts of the bone-active phosphonates which have the same general pharmacological properties as the acid form from which they are derived, and which are pharmaceutically acceptable. Pharmaceutically-acceptable salts include, for example, alkali metals (e.g., sodium and potassium), alkaline earth metals (e.g., calcium and magnesium), non-toxic heavy metals (e.g., stannous and indium), and ammonium and low molecular weight substituted ammonium (e.g., mono-, di- and triethanolamine) salts. Preferred compounds are the sodium, potassium, and ammonium salts. Pharmaceutically-acceptable esters include unsubstituted and substituted alkyl, aryl and phosphoryl esters. Nonlimiting examples of pharmaceutically-acceptable esters include, for example, isopropyl, tertiarybutyl, 2-chloroethyl, 2,2,2-trichloroethyl, 2,2,2-trifluoroethyl, p-toluenesulfonylethyl, glycyl, sarcosyl, benzyl, phenyl, 1,2-hexanoylglyceryl, p-nitrophenyl, 2,2 dimethyl-1,3-dioxolene-4-methyl, isopentenyl, O-carbomethoxyphenyl, piraloyloxymethylsalicylyl, diethylamidophosphoryl, pivaloyloxymethyl, acyloxymethyl, propionyloxymethyl, isobutyryloxymethyl, dodecyl, octadecyl, and isopropyloxymethyl.

Specific examples and definitions for substituents useful in the compounds of Formulas (1) through (6) are described in European Patent Publication 298,553, Ebetino, published January 11, 1989 (incorporated by reference herein). That application also describes phosphonoalkylphosphinates useful in the methods of this invention (wherein R is hydrogen or alkyl), and methods for making such compounds. Methods of making phosphonoalkylphosphinates are also described in European Patent Publication 298,555, Ebetino, published January 11, 1989.

Phosphonate compounds (wherein R is hydroxy) and methods for making such compounds, are described in the following patent documents, all incorporated by reference herein: U.S. Patent 3,553,314, Francis, issued January 5, 1971; U.S. Patent 3,683,080, Francis, issued August 8, 1972; U.S. Patent 3,846,420, Wollmann et al., issued November 5, 1974; U.S. Patent 3,899,496, Schindler et al., issued August 12, 1975; U.S. Patent 3,941,772, Ploger et al., issued March 2, 1976; U.S. Patent 3,957,160, Ploger et al., issued May 18, 1976; U.S. Patent 3,962,432, Schmidt-Dunker, issued June 8, 1976; U.S. Patent 3,979,385, Wollmann et al., issued September 7, 1976; U.S. Patent 3,988,443, Ploger et al., issued October 26, 1976; U.S. Patent 4,054,598, Blum et al., issued October 18, 1977; U.S. Patent 4,113,861, Fleisch et al., issued September 12, 1978; U.S. Patent 4,117,090, Ploger, issued September 26, 1978; U.S. Patent 4,134,969, Schmidt-Dunker, issued January 16, 1979; U.S. Patent 4,230,700, Francis, issued October 28, 1980; U.S. Patent 4,267,108, Blum et al., issued May 12, 1981; U.S. Patent 4,304,734, Jary et al., issued December 8, 1981; U.S. Patent 4,330,537, Francis, issued May 18, 1982; U.S. Patent 4,407,761, Blum et al., issued October 4, 1983; U.S. Patent 4,469,686, Andrews, issued September 4, 1984; U.S. Patent 4,578,376, Rosini, issued March 25, 1986; U.S. Patent 4,608,368, Blum et al., issued August 26, 1986; U.S. Patent 4,621,077, Rosini et al., issued November 4, 1986; U.S. Patent 4,687,767, Bosies et al., issued August 18, 1987; U.S. Patent 4,687,768, Benedict et al., issued October 18, 1987; U.S. Patent 4,711,880, Stahl et al., issued December 8, 1987; U.S. Patent 4,719,203, Bosies et al., issued January 12, 1988; U. S. Patent 4,868,164 to Ebetino, issued September 19, 1989; U. S. Patents 5,071,840 to Ebetino, et al, issued December 10, 1991, U.S. Patent 4,868,164, to Ebetino, et al., issued September 19, 1989; U.S. Patent 5,104,863, to Benedict, et al., issued April 14, 1992; U.S. Patent 4,267,108, to Blum et al., issued May 12, 1981; U.S.

Patent to Breliere, et al., issued May 24, 1988; U.S. Patent 4,876,247 to Barbier, et al., issued October 24, 1989; European Patent Application Publication No. 170,228, of Boehringer Mannheim GmbH, published February 5, 1986; European Patent Application Publication No. 298,553, of Ebetino, published January 11, 1989; U.S. 4,754,993, to Bosies, et al., issued November 15, 1988; U.S. 4,939,130 to Jaeggi, et al., issued July 3, 1990; U.S. 4,971,958 to Bosies, et al., issued November 20, 1990; WO 90/12017 to Dunn, et al., published October 18, 1990; WO 91/10646 to Youssefyeh, R., et al., published July 25, 1991; AU-A-26738/88 to Jaeggi, K. A., publication date June 15, 1989; AU-A-45467/89 of Ciba-Geigy, publication date May 31, 1990; U.S. 4,208,401, Bauman (assigned to Colgate-Palmolive Co.), issued June 17, 1980; DE 40 11 777, Jaeggi, K., disclosed October 18, 1990; U.S. Patent 4,927,814, Gall et al., issued May 22, 1990; U.S. Patent 4,990,503, Isomura et al., issued February 5, 1991; German Offenlegungsschrift 2,104,476, Worms, published August 17, 1972; German Offenlegungsschrift 2,343,147, Ploeger et al., published April 3, 1975; German Offenlegungsschrift 2,360,798, Worms et al., published June 26, 1975; German Offenlegungsschrift 2,513,966, Schmidt-Dunker, published October 7, 1976; German Offenlegungsschrift 2,541,981, Eimers et al., published March 24, 1977; German Offenlegungsschrift 3,334,211, Blum, published April 4, 1985, Japanese Patent Publication 78/59,674, Suzuki et al., published May 29, 1978; Japanese Patent Publication 79/135,724, Suzuki et al., published October 22, 1979; Japanese Patent Publication 80/98193, Suzuki et al., published July 25, 1980; European Patent Publication 88,359, Blum et al., published September 14, 1983; European Patent Publication 100,718, Breliere et al., published February 15, 1984; European Patent Publication 186,405, Benedict et al., published July 2, 1986; European Patent Publication 197,478, Bosies et al., published October 15, 1986; European Patent Publication 230,068, Benedict et al., published July 29, 1987; European Patent Publication 273,514, Ebetino et al., published July 6, 1988; European Patent Publication 274,158, Ebetino et al., published July 13, 1988; European Patent Publication 282,309, Sakamoto et al., published September 14, 1988; European Patent Publication 282,320, Isomura et al., published September 14, 1988; PCT Patent Publication 87/03598, Binderup et al., published June 18, 1987; and PCT Patent Publication 88/00590, Gall et al., published January 28, 1988. Additionally, quaternary nitrogen containing and thiol-substituted phosphonate compounds are described in the following documents, all incorporated by reference herein: U.S.S.N. 07/890,885, Ebetino, et al., filed May 29, 1992; U.S.S.N. 07/891,487 Ebetino, et al., filed May 29, 1992; U.S.S.N. 07/891,335, Francis, et al., filed May 29, 1992; U.S.S.N. 07/891,490, Kaas, et al., filed May 29, 1992; U.S.S.N. 07/890,886, Kaas, et al., filed May 29, 1992; U.S.S.N. 07/891,309, Francis et al., filed May 29, 1992.

Preferred bone-active phosphonates useful in the methods of this invention include:

2-(3-pyridyl)-1-hydroxyethane-1,1-bisphosphonic acid;
3-(N-pentyl-N-methyl)-amino-1-hydroxypropane-1,1-bisphosphonic acid;
4-amino-1-hydroxybutane-1,1-bisphosphonic acid;
N-cycloheptylaminomethane bisphosphonate;
3-(N,N-dimethyl)-amino-1-hydroxypropane-1,1-bisphosphonic acid;
N-(2'-(3'-methyl)-pyridinyl)aminomethane phosphonomethylphosphinic acid;
N-(2'-(5'-methyl)-pyridinyl)aminometane phosphonomethylphosphinic acid;
N-(2'-(3'-methyl)-piperidinylidene)aminomethane phosphonomethylphosphinic acid;
N-(2'-(5'-methyl)-piperidinylidene)aminomethane phosphonomethylphosphinic acid;
2-(2'-pyridinyl)ethane-1-phosphono-1-methylphosphinic acid;
2-(2'-piperidinyl)ethane-1-phosphono-1-methylphosphinic acid;
N-(2'-(3'-methyl)-piperidinylidene)aminomethane phosphonobutylphosphinic acid;
S-(2'-pyridinyl)thiomethane phosphonomethylphosphinic acid;
2-(2-pyridyl)-1-hydroxyethane-1-phosphono-1-methylphosphinic acid;
2-(3-pyridyl)-1-hydroxyethane-1-phosphono-1-methylphosphinic acid;
2-(N-imidazoyl)-1-hydroxyethane-1-phosphono-1-methylphosphinic acid;
3-(N-pentyl-N-methylamino)-1-hydroxypropane-1-phosphono-1-methylphosphinic acid;
4-amino-1-hydroxybutane-1-phosphono-1-methylphosphinic acid;
3-(N-pyrollidino)-1-hydroxypropane-1-phosphono-1-methylphosphinic acid;
2-(6-pyrolopyridine)-1-hydroxyethane-1-phosphono-1-methylphosphinic acid;
4-amino-1-hydroxybutane-1,1-bisphosphonic acid;
2-(2-pyridyl)-1-hydroxyethane-1,1-bisphosphonic acid;
N-(2-(5-amino)-pyridyl)-aminomethane bisphosphonic acid;
N-(2-(5-chloro)-pyridyl)-aminomethane bisphosphonic acid;
N-(2-(3-picolyl))-aminomethane bisphosphonic acid;
N-(2-(4-picolyl))-aminomethane bisphosphonic acid;
N-(2-(5-picolyl))-aminomethane bisphosphonic acid;
N-(2-(6-picolyl))-aminomethane bisphosphonic acid;
N-(2-(3,4-lutidine))-aminomethane bisphosphonic acid;

N-(2-pyrimidyl)-aminomethane bisphosphonic acid;
N-(2-pyridyl)-2-aminoethane-1,1-bisphosphonic acid;
2-(2-pyridyl)-ethane-1,1-bisphosphonic acid;
2-(3-pyridyl)-ethane-1,1-bisphosphonic acid;
2-(4-pyridyl)-ethane-1,1-bisphosphonic acid;
2-(2-(3-picolyl))-oxaethane-1,1-bisphosphonic acid;
2-(N-imidazoyl)-1-hydroxyethane-1,1-bisphosphonic acid;
3-(N-pyrollidino)-1-hydroxypropane-1,1-bisphosphonic acid;
2-(6-pyrolopyridine)-1-hydroxyethane-1,1-bisphosphonic acid;

and pharmaceutically-acceptable salts and esters thereof.

Particularly, preferred bone-active phosphonates useful in the methods of this invention include:

2-(3-pyridyl)-1-hydroxyethane-1,1-bisphosphonic acid;
4-amino-1-hydroxybutane-1,1-bisphosphonic acid;
2-(N-imidazoyl)-1-hydroxyethane-1,1-bisphosphonic acid;
3-(N-pentyl-N-methyl)-amino-1-hydroxypropane-1,1-bisphosphonic acid;
N-cycloheptylaminomethane bisphosphonate;
3-(N,N-dimethyl)-amino-1-hydroxypropane-1,1-bisphosphonic acid;
3-(N-pyrollidino)-1-hydroxypropane-1,1-bisphosphonic acid;
2-(6-pyrolopyridine)-1-hydroxyethane-1,1-bisphosphonic acid;
2-(2-hydroxy-2,2-diphosphonoethyl)-1,1-dimethylpiperidinium iodide salt;
3-(2-hydroxy-2,2-diphosphonoethyl)-1-methylpyridinium iodide;
3-(2-hydroxy-2,2-diphosphonoethyl)-1-methylpyridinium hydroxide;
3-(2,2-diphosphonoethyl)-1-ethylpyridinium chloride;
3-(2,2-diphosphonoethyl)-1-(2-mercaptoethyl)pyridinium chloride;
2-(2-hydroxy-2,2-diphosphonoethyl)-1-methylpyridinium hydroxide;
3-(3-hydroxy-3,3-diphosphonopropyl)-1-methylpyridinium hydroxide;
3-(2,2-diphosphono-2-hydroxyethyl)-1,1-dimethylpiperidinium iodide salt;
3-(2,2-diphosphonoethyl)-1-heptylpyridinium chloride;
3-(2,2-diphosphonoethyl)-1-methylpyridinium chloride;
3-(2,2-phosphonomethylphosphinoethyl)-1-methylpyridinium iodide;
3-(2-phosphono-2-sulfonoethyl)-1-methylpyridinium chloride;
3-(2-carboxy-2-phosphonoethyl)-1-methylpyridinium chloride;
2-diphosphonomethyl-1,1-dimethylpiperidinium chloride;
3-diphosphonomethyl-1,1-dimethylpiperidinium chloride;
4-diphosphonomethyl-1,1-dimethylpiperidinium chloride;
2-(2,2-diphosphonoethyl)-1,1-dimethylpiperidinium chloride;
3-(2,2-diphosphonoethyl)-1,1-dimethylpiperidinium chloride;
4-(2,2-diphosphonoethyl)-1,1-dimethylpiperidinium chloride;
2-(2,2-diphosphonoethyl)-1-methyl-1-(2-mercaptoethyl)piperidinium chloride;
3-(2,2-diphosphonoethyl)-1-methyl-1-(2-mercaptoethyl)piperdinium chloride;
4-(2,2-diphosphonoethyl)-1-methyl-1-(2-mercaptoethyl)piperidinium chloride;
2-[2,2-diphosphono-1-(2-mercaptoethyl)ethyl]-1,1-dimethylpiperidinium chloride;
3-[2,2-diphosphono-1-(3-mercaptopropyl)ethyl]-1,1-dimethylpiperidinium chloride;
4-[2,2-diphosphono-1-(2-acetylthioethyl)ethyl]-1,1-dimethylpiperidinium chloride;
2-(2,2-diphosphono-2-hydroxyethyl)-1,1-dimethylpiperidinium chloride;
3-(2,2-diphosphono-2-hydroxyethyl)-1,1-dimethylpiperidinium chloride;
4-(2,2-diphosphono-2-hydroxyethyl)-1,1-dimethylpiperidinium chloride;
2-(2,2-diphosphono-2-hydroxyethyl)-1,1,3-trimethylpiperidinium chloride;
2-(2,2-diphosphono-2-hydroxyethyl)-1,1,5-trimethylpiperidinium chloride;
2-(2,2-diphosphonoethyl)-1,1,3-trimethylpiperidinium chloride;
2-(2,2-diphosphonoethyl)-1,1,5-trimethylpiperidinium chloride;
2-(3,3-diphosphonopropyl)-1,1-dimethylpiperidinium chloride;
3-(3,3-diphosphonopropyl)-1,1-dimethylpiperidinium chloride;
4-(3,3-diphosphonopropyl)-1,1-dimethylpiperidinium chloride;
2-(3,3-diphosphono-3-hydroxypropyl)-1,1-dimethylpiperidinium chloride;
3-(3,3-diphosphono-3-hydroxypropyl)-1,1-dimethylpiperidinium chloride;

4-(3,3-diphosphono-3-hydroxypropyl)-1,1-dimethylpiperidinium chloride;
2-(2,2-diphosphonopropyl)-1,1-dimethylpiperidinium chloride;
3-(2,2-diphosphonopropyl)-1,1-dimethylpiperidinium chloride;
4-(2,2-diphosphonopropyl)-1,1-dimethylpiperidinium chloride;
2-(2,2-diphosphono-2-aminoethyl)-1,1-dimethylpiperidinium chloride;
3-(2,2-diphosphono-2-aminoethyl)-1,1-dimethylpiperidinium chloride;
4-(2,2-diphosphono-2-aminoethyl)-1,1-dimethylpiperidinium chloride;
2-(2,2-diphosphono-2-aminoethyl)-1,1,3-trimethylpiperidinium chloride;
2-(2,2-diphosphono-2-aminoethyl)-1,1,3-trimethylpiperidinium chloride;
3-(2,2-diphosphono-2-aminoethyl)-1,1,5-trimethylpiperidinium chloride;
2-(2,2-diphosphono-2-(methylamino)ethyl)-1,1,-dimethylpiperidinium chloride;
2-(4,4-diphosphono-4-hydroxybutyl)-1,1,3-trimethylpiperidinium chloride;
2-(4,4-diphosphono-4-hydroxybutyl)-1,1-dimethylpiperidinium chloride;
2-(2,2-diphosphono-2-hydroxyethyl)-3-carboxy-1,1-dimethylpiperidinium chloride;
2-(2,2-diphosphono-2-hydroxyethyl)-5-carboxy-1,1-dimethylpiperidinium chloride;
2-(2,2-diphosphonoethyl)-1-methylpyrimidinium chloride;
4-(2,2-diphosphonoethyl)-1-methylpyrimidinium chloride;
2-(2,2-diphosphono-2-hydroxyethyl)-1-methylpyrimidinium chloride;
4-(2,2-diphosphono-2-hydroxyethyl)-1-methylpyrimidinium chloride;
2-(3,3-diphosphonopropyl)-1-methylpyrimidinium chloride;
4-(3,3-diphosphonopropyl)-1-methylpyrimidinium chloride;
2-(3,3-diphosphono-1-hydroxypropyl)-1-methylpyrimidinium chloride;
4-(3,3-diphosphono-1-hydroxypropyl)-1-methylpyrimidinium chloride;
2-(2,2-diphosphono-2-aminoethyl)-1-methylpyrimidinium chloride;
3-[(diphosphonomethyl)oxo]-1,1-dimethylpiperidinium chloride;
4-[(diphosphonomethyl)oxo]-1,1-dimethylpiperidinium chloride;
3-[(2,2-diphosphonoethyl)oxo]-1,1-dimethylpiperidinium chloride;
4-[(2,2-diphosphonoethyl)oxo]-1,1-dimethylpiperidinium chloride;
3-[(diphosphonomethyl)thio]-1,1-dimethylpiperidinium chloride;
4-[(diphosphonomethyl)thio]-1,1-dimethylpiperidinium chloride;
3-(2-hydroxy-2,2-diphosphonoethyl)-1-methylpyridinium iodide;
3-(2-hydroxy-2,2-diphosphonoethyl)-1-methylpyridinium hydroxide;
3-(2,2-diphosphonoethyl)-1-(2-mercaptoethyl)pyridinium chloride;
2-(2-hydroxy-2,2-diphosphonoethyl)-1,1-dimethylpiperidinium iodide salt;
3-(2,2-diphosphono-2-hydroxyethyl)-1,1-dimethylpiperidinium iodide salt;
3-(2,2-diphosphonoethyl)-1-heptylpyridinium chloride;
3-(2,2-diphosphonoethyl)-1-methylpyridinium chloride;
2-(2,2-diphosphonoethyl)-1,1-dimethylpiperidinium chloride;
3-(2,2-diphosphonoethyl)-1,1-dimethylpiperidinium chloride;
4-(2,2-diphosphonoethyl)-1,1-dimethylpiperidinium chloride;
2-(2,2-diphosphono-2-hydroxyethyl)-1,1-dimethylpiperidinium chloride;
3-(2,2-diphosphono-2-hydroxyethyl)-1,1-dimethylpiperidinium chloride;
4-(2,2-diphosphono-2-hydroxyethyl)-1,1-dimethylpiperidinium chloride;
2-(2,2-diphosphono-2-hydroxyethyl)-1,1,3-trimethylpiperidinium chloride;
2-(2,2-diphosphono-2-hydroxyethyl)-1,1,5-trimethylpiperidinium chloride;
2-[2,2-diphosphono-1-(2-mercaptoethyl)ethyl]-1,1-dimethylpiperidinium chloride;
3-[2,2-diphosphono-1-(3-mercaptopropyl)ethyl]-1,1-dimethylpiperidinium chloride;
2-(2,2-diphosphonoethyl)-1-methyl-1-(2-mercaptoethyl)piperidinium chloride;
3-(2,2-diphosphonoethyl)-1-methyl-1-(2-mercaptoethyl)piperidinium chloride;
4-(2,2-diphosphonoethyl)-1-methyl-1-(2-mercaptoethyl)piperidinium chloride;
3-(2-hydroxy-2,2-diphosphonoethyl)-1-methylpyridinium iodide;
3-(2-hydroxy-2,2-diphosphonoethyl)-1-methylpyridinium hydroxide;
3-(2,2-diphosphonoethyl)-1-(2-mercaptoethyl)pyridinium chloride;
2-[2,2-diphosphono-1-(2-mercaptoethyl)ethyl]-1,1-dimethylpiperidinium chloride;
3-[2,2-diphosphono-1-(3-mercaptopropyl)ethyl]-1,1-dimethylpiperidinium chloride;
2-(2,2-diphosphonoethyl)-1-methyl-1-(2-mercaptoethyl)piperidinium chloride;
3-(2,2-diphosphonoethyl)-1-methyl-1-(2-mercaptoethyl)piperidinium chloride;
N-(4-hydroxy-4,4-diphosphonobutyl)-N,N,N-trimethyl ammonium iodide;

N-(3-hydroxy-3,3-diphosphonopropyl)-N,N-dimethyl-N-pentyl ammonium iodide;
N,N-dimethyl-N-(4,4-diphosphonobutyl)-N-(2-(3-piperidinyl)ethyl) ammonium chloride;
[(5-[mercaptomethyl]-2-piperidinyl)methylene]bis[phosphonic acid;
[(5-mercaptomethyl-3-piperidinyl]methylene]bis[phosphonic acid;
[(5-mercapto-2-piperidinyl)methylene]bis[phosphonic acid;
[(5-[4-mercaptobutyl]-2-piperidinyl)methylene]bis[phosphonic acid;
[(5-mercapto-3-piperidinyl)methylene]bis[phosphonic acid;
[(5-[5-mercaptopentyl]-3-piperidinyl)methylene]bis[phosphonic acid;
[(5-[2-mercaptoethyl]-4-piperidinyl)methylene]bis[phosphonic acid;
[(5-mercapto-4-piperidinyl)methylene]bis[phosphonic acid;
[2-(5-mercapto-2-piperidinyl)ethylidene]bis[phosphonic acid];
[2-(5-[3-mercaptopropyl]-2-piperidinyl)ethylidene]bis[phosphonic acid];
[2-(5-mercapto-3-piperidinyl)ethylidene]bis[phosphonic acid];
[2-(5-mercapto-4-piperidinyl)ethylidene]bis[phosphonic acid];
[2-(5-[4-mercaptobutyl]-2-piperidinyl)ethylidene]bis[phosphonic acid];
[2-(5-mercaptomethyl-3-piperidinyl)ethylidene]bis[phosphonic acid];
[(2-[5-mercapto-2-piperidinyl]-1-hydroxy)ethylidene]bis[phosphonic acid];
[(2-[5-(3-mercaptopropyl)-2-piperidinyl]-1-hydroxy)ethylidene]bis[phosphonic acid];
[(2-[5-mercapto-3-piperidinyl]-1-hydroxy)ethylidene]bis[phosphonic acid];
[(2-[5-(2-mercaptoethyl)-3-piperidinyl]-1-hydroxy)ethylidene]bis[phosphonic acid];
[(2-[5-mercapto-4-piperidinyl]-1-hydroxy)ethylidene]bis[phosphonic acid];
[(2-[5-mercaptomethyl-4-piperidinyl]-1-hydroxy)ethylidene]bis-[phosphonic acid];
[(2-[5-mercaptomethyl-3-methyl-2-piperidinyl]-1-hydroxy)ethylidene]bis[phosphonic acid];
[(2-[5-mercapto-3-methyl-2-piperidinyl]-1-hydroxy)ethylidene]bis[phosphonic acid];
[(2-[3-mercaptomethyl-5-methyl-2-piperidinyl]-1-hydroxy)-ethylidene]bis[phosphonic acid];
[2-(5-mercaptomethyl-3-methyl-2-piperidinyl)-ethylidene]bis[phosphonic acid];
[2-(3-mercaptomethyl-5-methyl-2-piperidinyl)ethylidene]bis[phosphonic acid];
[3-[5-(mercaptomethyl)-2-piperidinyl]propylidene]bis[phosphonic acid];
[3-[5-(mercaptomethyl)-3-piperidinyl]propylidene]bis[phosphonic acid];
[3-[5-(mercaptomethyl)-4-piperidinyl]propylidene]bis[phosphonic acid];
[3-[5-(mercaptomethyl)-2-piperidinyl]-1-hydroxypropylidene]bis[phosphonic acid];
[3-[5-mercapto-3-piperidinyl]-1-hydroxypropylidene]-bis[phosphonic acid];
[3-[5-(4-mercaptobutyl)-4-piperidinyl]-1-hydroxypropylidene]-bis[phosphonic acid];
[2-(3-mercaptomethyl-5-methyl-2-pyridinyl)ethylidene]-bis[phosphonic acid];
[2-(5-[3-mercaptopropyl]-2-methyl-2-piperidinyl)ethylidene]bis[phosphonic acid];
[(2-[5-(2-mercaptopropyl)-2-piperidinyl]-1-amino)ethylidene]bis[phosphonic acid];
[(2-[5-(3-mercaptopropyl)-3-piperidinyl]-1-amino)ethylidene]bis[phosphonic acid];
[2-(5-[3-mercaptopropyl]-4-piperidinyl)-1-aminoethylidene]bis[phosphonic acid];
[(2-[3-methyl-5-(3-mercaptopropyl)-2-piperidinyl]-1-hydroxy)ethylidene]bis[phosphonic acid];
[(2-[3-amino-5-(3-mercaptopropyl)-2-piperidinyl]-1-hydroxy)ethylidene]bis[phosphonic acid];
[2-[5-mercapto-2-(1,4-diazinyl)]ethylidene]bis[phosphonic acid];
[2-[5-(3-mercaptopropyl)-2-(1,4-diazinyl)]ethylidene]bis[phosphonic acid];
[2-[5-(3-mercaptopropyl)-2-(1,4-diazinyl)]-1-hydroxyethylidene]bis[phosphonic acid];
[2-[5-mercapro-2-(1,4-diazinyl)]-1-hydroxyethylidene]bis[phosphonic acid];
[2-[5-mercapto-2-(1,3-diazinyl)]ethylidene]bis[phosphonic acid];
[2-[5-(3-mercaptopropyl)-2-(1,3-diazinyl)]ethylidene]bis[phosphonic acid];
[2-[5-(3-mercaptopropyl)-2-(1,3-diazinyl)]-1-hydroxyethylidene]bis[phosphonic acid];
[2-[5-mercapto-2-(1,3-diazinyl)]-1-hydroxyethylidene]bis[phosphonic acid];
[(5-[3-mercaptopropyl]-2-piperidinyl)aminomethylene]bis[phosphonic acid];
[(5-mercapto-2-piperidinyl)aminomethylene]bis[phosphonic acid];
[(5-[3-mercaptopropyl]-3-piperidinyl)aminomethylene]bis[phosphonic acid];
[(5-mercapto-3-piperidinyl)aminomethylene]bis[phosphonic acid];
[(5-mercapto-4-piperidinyl)aminomethylene]bis[phosphonic acid];
[(5-[3-mercaptopropyl]-4-piperidinyl)aminomethylene]bis[phosphonic acid];
[(5-mercapto-3-methyl-2-piperidinylidene)aminomethylene]bis[phosphonic acid];
[(5-[3-mercaptopropyl]-3-methyl-2-piperidinylidene)aminomethylene]bis[phosphonic acid];
[2-(5-mercapto-3-methyl-2-piperidinylidene)aminoethylene]bis[phosphonic acid];
[2-(5-[3-mercaptopropyl]-3-methyl-2-piperidinylidene)aminomethylene]bis[phosphonic acid];

[(5-mercapto-2-piperidinylidene)aminomethylene]bis[phosphonic acid];
[(5-[3-mercaptopropyl]-2-piperidinylidene)aminomethylene]bis[phosphonic acid];
[2-(5-mercapto-2-piperidinylidene)aminoethylene]bis[phosphonic acid];
[(5-[3-mercaptopropyl]-2-piperidinylidene)aminomethylene]bis[phosphonic acid];
[(5-[3-mercaptopropyl]-2-[1,4-diazinylidene])aminomethylene]bis[phosphonic acid];
[(5-[3-mercaptopropyl]-2-(1,3-diazinylidene])aminomethylene]bis[phosphonic acid];
[(4-[3-mercaptopropyl]-2-[1,3,5-triazinylidene])aminomethylene]bis[phosphonic acid];
N-(2'-(1',3'-diazinylidene))-aminomethane diphosphonic acid; and

the pharmaceutically-acceptable salts and esters thereof.

Use in the manufacture for increasing bone mass

A use for increasing bone mass in a human or other mammal subject afflicted with osteoporosis, comprising a thirty (30) day treatment period, comprised of a high potency phosphonate compound administration regimen wherein

(a) said high potency phosphonate administration regimen comprises the systemic administration to said subject of a high potency phosphonate compound at a level of from 0.00001 mgP/kg to 0.1 mgP/kg per day that said high potency phosphonate compound is administered, provided that said level of the high potency phosphonate compound is administered at least 1 day of every said thirty (30) day treatment period; and wherein
(b) said thirty (30) day treatment period may be followed by a rest period of at least one day.

Accordingly, the high potency phosphonate must be given at a level of 0.00001 mgP/kg-0.1 mgP/kg per day at least one day of every thirty (30)-day treatment period. However, said high potency phosphonate may be given every day of said thirty (30)-day treatment period, or every other day of said thirty (30)-day treatment period, or every third day, or every fourth day, or every fifth day, or every sixth day of said thirty (30)-day treatment period, or every seventh day of said thirty (30)-day treatment period. The administration of a high potency phosphonate may or may not be followed by a rest period of at least one (1) day. As long as the high potency phosphonate is given at a dose of 0.00001 mgP/kg - 0.1 mgP/kg per day, it may be given at a different dose within the 0.00001 mgP/kg - 0.1 mgP/kg range on different days, so long as it is given on at least one day of said thirty (30)-day treatment period.

The term "rest period", as used herein, is the period of time when no high potency phosphonate is administered.

The term "systemic administration", as used herein, refers to the administration of the phosphonate compound wherein said phosphonate compound is delivered to the receptor locus, i.e. the bone, via the systemic circulation. The more rapid form of systemic administration is via injection (parenterally), i.e. subcutaneously, intramuscularly and intravenously. Systemic administration via oral dosing is accomplished by absorption of the phosphonate from the stomach into the systemic circulation. While oral dosing is convenient, it is not as efficient as parenteral administration. For this reason, oral dosing of phosphonate compounds is typically ten to one hundred fold higher than dosing of phosphonate compounds by injection.

The terms "low potency", "medium potency", and "high potency" are used to describe the bone antiresorptive capacity of the phosphonate. For example, low potency phosphonates have a subcutaneous LED of 1.0 - 0.5; medium potency phosphonates have a subcutaneous LED of 0.5 - 0.01, and high potency phosphonates have a subcutaneous LED of 0.01 - 0.0001.

The term "high potency", as used herein, generally refers to those phosphonate compounds having a nitrogen heteroatom. The importance of the nitrogen heteroatom and its role in establishing potency is described in Sietsema, W. K., et al., "Anti-Resorptive Dose-Response Relationships Across Three Generations of Bisphosphonates", XV(9) Drugs Exptl. Clin. Res. 389-396 (1989).

The potency of a particular phosphonate can be expressed in terms of its "LED" or "least effective dose", which is the minimum dose of phosphonate expressed in mg P/kg that is effective, by itself, to cause a significant inhibition of bone resorption, i.e. the anti-resorptive dose. The specific LEDs of the phosphonates will vary depending upon their chemical composition, and their method of administration (i.e., oral or parenteral). The lower the LED, the more potent the anti-resorptive effect of the phosphonate. Therefore, according to the methods of the invention, the high potency phosphonates can be administered in low doses, that are below the doses administered to cause the anti-resorptive effect described hereinbefore, and on a fewer number of days in said thirty (30)-day treatment period. Conversely, the higher the LED, the less potent the phosphonate and the more often it would need to be administered.

The "low potency phosphonates" include, but are not limited to, 1-hydroxy ethane-1,1-bisphosphonate, [[(4-chlorophenyl)thio]methylene]bisphosphonic acid; and dichloromethane bisphosphonate. The "medium potency phosphonates" include, but are not limited to, (3-amino-1-hydroxypropylidene)-1,1-bisphosphonate. The "high potency phosphonates" include, but are not limited to, N-cycloheptylaminomethane bisphosphonate, 2-(3-pyridinyl)-1-hydroxyethane bisphosphonate; 4-amino-1-hydroxybutane-1,1-bisphosphonate, 3-(2,2-diphosphonoethyl)-1-methylpyridinium chloride, 3-(2,2-diphosphonoethyl)-1-(2-mercapto ethyl)pyridinium chloride, 3-(2-hydroxy-2,2-diphosphonoethyl)-1-methylpyridiniumiodide disodium salt; 3-(N-pentyl-N-methyl)-amino-1-hydroxypropane-1,1-bisphosphonic acid; 2-(N-imidazoyl)-1-hydroxyethane-1,1-bisphosphonic acid; 3-(N,N-dimethyl)-amino-1-hydroxy propane-1,1-bisphosphonate.

In particular, the LEDs for the bone-active phosphonates may be determined using any of several art-recognized in vivo models. One such model is the thyroparathyroidectomized ("TPTX") rat model. In this model, compounds are evaluated for in vivo bone resorption inhibition potency, by measuring their ability to inhibit the increase of serum calcium levels caused by administration of parathyroid hormone in rats whose parathyroid gland has been removed. This model is described in Russell et al., 6 Calcified Tissue Research 183 (1970); Muhlbauer et al., 5 Mineral Electrolite Metabolism 296 (1981); U.S. Patent 4,761,406, Flora et al., issued August 2, 1988; and European Patent Publication 298,553, Ebetino, published January 11, 1989; all of which are incorporated by reference herein.

Another model is the "Schenk Model", which measures the effects of bone-active phosphonates on bone growth in young rats. This model is described in Schenk et al., 11 Calcif. Tissue Res. 196 (1973); Shinoda et al., 35 Calcif. Tissue Int. 87 (1983); U.S. Patent 4,761,406, Flora et al., issued August 2, 1988; and European Patent Publication 298,553, Ebetino, published January 11, 1989; all of which are incorporated by reference herein.

Another model is the "ovariectomized" or "OVX" rat model, which measures the ability of bone-active phosphonates to prevent loss of bone in female rats induced by ovariectomy. This model is described in Wronski et al., 125 Endocrinology 810 (1989), incorporated by reference herein.

The LEDs, as determined by the "Schenk Model", for parenteral dosing of representative phosphonates are: 1.0 mg P/kg, for 1-hydroxyethane-1,1-bisphosphonic acid; 0.5 mg P/kg, for dichloromethane bisphosphonic acid: 0.03 mg P/kg, for 3-amino-1-hydroxypropane-1,1-bisphosphonic acid; 0.001 mg P/kg, for 4-amino-1-hydroxybutane-1,1-bisphosphonic acid; 0.1 mg P/kg, for 6-amino-1-hydroxyhexane-1,1-bisphosphonic acid; 0.01 mg P/kg, for N-(2-pyridyl) aminomethane-1,1-bisphosphonic acid; 0.0003 mg P/kg, for 2-(3-pyridyl)-1-hydroxyethane-1,1-bisphosphonic acid; 0.0003 mg P/kg, for N-cycloheptyl-aminomethanebisphosphonic acid; 0.0001 mg P/kg, for 3-(N-pentyl-N-methylamino)-1-hydroxypropane-1,1-bisphosphonic acid; 0.01 mg P/kg, for 3-(dimethylamino)-1-hydroxypropane-1,1-bisphosphonic acid; 0.01 mg P/kg, for 3-(N-pyrollidino)-1-hydroxypropane-1,1-bisphosphonic acid; and 0.3 mg P/kg for S-(p-chlorophenyl)thiomethanebisphosphonic acid. The LEDs for oral dosing would be higher, depending upon the systemic absorption of the phosphonate. Typically, absorption from oral administration is from about 1% to about 10%. Thus, oral LEDs are typically about ten- to one hundred-fold higher than the parenteral LEDs.

As used herein, the term "mg P/kg" refers to the amount of compound, expressed as milligrams phosphorus in the compound, per kilogram weight of the subject to be treated. Because the phosphonates vary in molecular weight, expressing the amount administered in mg P/kg normalizes the comparison between bisphosphonates of varying potencies. In order to determine the mg P/kg administered to a patient according to the methods of this invention, the following conversion formula is used:

$$\text{mg/kg compound administered} = \frac{\text{mg P}}{\text{kg}} \times \frac{\text{molecular weight of the drug}}{\text{molecular weight of two phosphorus atoms}}$$

For example, 2-(3-pyridinyl)-1-hydroxyethane-1,1-bisphosphonate has a molecular weight of 350. Two phosphorus atoms have a molecular weight of 62. Thus, if a patient is dosed at 0.01 mg/kg of the compound, then about 0.002 mg P/kg was administered.

This invention comprises the use for the treatment of osteoporosis at all stages of the disorder. Since osteoporosis is an ongoing process of bone loss, rather than a disorder having a discrete beginning- or end-point, "treatment", as referred to herein, consists of any method which stops, slows, or reverses the process of bone loss which occurs in osteoporosis.

This invention comprises the use for the treatment of osteoporosis in subjects who have already lost skeletal mass (herein referred to as "established osteoporosis"). Such methods of this invention for the treatment of established osteoporosis preferably comprise administering the actives for a period of time sufficient to achieve an increase in the net skeletal mass of said subject. The increase in mass may be in cortical bone, trabecular bone, or both. Preferably, the net skeletal mass is increased by about 1% to 3% per year.

The specific period of time sufficient to achieve an increase in the net skeletal mass of the subject may depend on a variety of factors. Such factors include, for example, the specific actives employed, the amount of actives administered, the age and sex of the subject, the specific disorder to be treated, concomitant therapies employed (if any), the general physical health of the subject (including the presence of other disorders), the extent of bone loss in the individual, and the nutritional habits of the individual.

The therapeutic regimen utilizing the use of this invention are preferably continued for at least about twelve months. Of course, a therapeutic regimen may be continued indefinitely, according to sound medical practice. Preferably the subject is treated until a net skeletal mass is obtained commensurate with reduced fracture risk as assessed by the

patient's physician.

In the use of this invention, "administering" refers to any method which, in sound medical practice, delivers the actives used in this invention to the subject to be treated in such a manner so as to be effective in the building of bone. The actives may be administered by any of a variety of known methods of administration, e.g., orally, dermatomucosally (for example, dermally, sublingually, intranasally, and rectally), parenterally (for example, by subcutaneous injection, intramuscular injection, intra-articular injection, intravenous injection), and by inhalation. Thus, specific modes of administration include, but are not limited to, for example, oral, transdermal, mucosal, sublingual, intramuscular, intravenous, intraperitoneal, subcutaneous administration, and topical application.

A preferred use for the treatment of osteoporosis includes an initial diagnostic step, to determine the presence of the disorder. Thus, a preferred use of this invention comprises the steps of performing a diagnosis on a human subject for the detection of osteoporosis and, upon obtaining a positive result from said diagnosis, administering the actives according to the methods of this invention. For such a use for the treatment of postmenopausal female subjects prior to significant bone loss, said initial diagnostic step comprises performing a diagnostic test for determining postmenopausal bone loss. Such methods are well known in the art, and include determination of the bone mass and rate of bone remodeling. The rate of bone remodeling can be determined by measurement of biochemical markers. See, Hui, et al., "The Contribution of Bone Loss to Postmenopausal Osteoporosis," 1 Osteoporosis Int. 30 (1990), incorporated by reference herein.

Suitable diagnostics for the detection of established osteoporosis are also well known in the art. Such methods include the measurement of the radiodensity of skeletal radiographs, quantitative computerized tomography, single energy photon absorptiometry, and dual-energy photon absorptiometry. Diagnostic techniques among those useful herein are described in W. A. Peck et al., Physician's Resource Manual on Osteoporosis (1987), published by the National Osteoporosis Foundation (incorporated by reference herein).

Dosage Forms:

As stated hereinbefore, the subcutaneous absorption of the high potency phosphonate compound can be as much as one-hundred fold greater than the oral absorption of a high potency bisphosphonate compound. Therefore, the dosage form is important to the methods of this invention. The phosphonate compound may be administered in any of a variety of pharmaceutically-acceptable compositions. Such compositions may comprise an active and a pharmaceutically-acceptable excipient. Pharmaceutically-acceptable excipients include, for example, solid or liquid fillers, diluents, lubricants, binders, disintegrants, solvents, or encapsulating substances, and mixtures thereof, that are suitable for administration to a human or lower animal. The term "compatible", as used herein, means that the components of the pharmaceutical composition are capable of being commingled with the actives, and with each other, in a manner such that there is no interaction which would substantially reduce the pharmaceutical efficacy of the pharmaceutical composition under ordinary use situations. Pharmaceutically-acceptable excipients must, of course, be of sufficiently high purity and sufficiently low toxicity to render them suitable for administration to the human or lower animal being treated.

Some examples of the substances which can serve as pharmaceutical excipients are: sugars, such as lactose, glucose and sucrose; starches, such as corn starch and potato starch; cellulose and its derivatives, such as sodium carboxymethylcellulose, ethyl cellulose, cellulose acetate; powdered tragacanth; malt; gelatin; talc; stearic acid; magnesium stearate; vegetable oils, such as peanut oil, cottonseed oil, sesame oil, olive oil, corn oil and oil of theobroma; polyols such as propylene glycol, glycerin, sorbitol, mannitol, and polyethylene glycol; agar; alginic acid; pyrogen-free water; isotonic saline; phosphate buffer solutions; wetting agents and lubricants such as sodium lauryl sulfate; coloring agents; flavoring agents; and preservatives. Other compatible pharmaceutical additives and actives may be included in the pharmaceutically-acceptable excipients for use in the compositions of the present invention.

The choice of a pharmaceutically-acceptable excipients to be used in conjunction with the active is determined by the way the active is to be administered. If the active is to be injected, the preferred pharmaceutical excipient is sterile water, physiological saline, or mixtures thereof. The phosphonate active ingredient can be injected subcutaneously, intramuscularly or intravenously. The pH of such parenteral compositions is preferably adjusted to about 7.4. Preferably, injectable dosage forms of the high potency phosphonate comprise from 0.00001 mgP/kg per day to 0.01 mgP/kg per day of the high potency phosphonate.

If the active is to be administered topically, the phosphonate compounds is blended with a cream, gel or paste and applied to the skin as a patch.

The pharmaceutically-acceptable excipients employed in conjunction with the actives is used at a concentration sufficient to provide a practical size to dosage relationship. The pharmaceutically-acceptable excipients, in total, may comprise from about 0.1% to about 99.9% by weight of the pharmaceutical compositions of the present invention, preferably from about 50% to about 99.0%, and most preferably from about 75% to about 99.1%.

A preferred method of administering a phosphonate compound is orally, in a unit-dosage form (i.e., a dosage form containing an amount of active suitable for administration in one single dose, according to sound medical practice). Preferred unit dosage forms for phosphonate compounds include tablets, capsules, suspensions, and solutions, comprising a safe and effective amount of active. Pharmaceutically-acceptable excipients suitable for the preparation of unit dosage forms for oral administration are well known in the art. Their selection will depend on secondary considerations like taste, cost, shelf stability, which are not critical for the purposes of the present invention, and can be made without difficulty by a person skilled in the art. Preferably, oral unit dosage forms of a high potency phosphonate comprise from 0.001 mgP/kg oral per day to 0.1 mgP/kg oral per day of the high potency phosphonate.

Kits:

This invention also provides kits for conveniently and effectively implementing the use of this invention. Such kits comprise one or more unit doses of a phosphonate compound, and a means for facilitating compliance with methods of this invention. Such kits provide a convenient and effective means for assuring that the subject to be treated takes the appropriate active in the correct dosage in the correct manner. The compliance means of such kits includes any means which facilitates administering the actives according to a method of this invention. Such compliance means includes instructions, packaging, and dispensing means, and combinations thereof. Examples of packaging and dispensing means are well known in the art, including those described in U.S. Patents 4,761,406, Flora et al., issued August 2, 1988; and U.S. Patent 4,812,311, Uchtman, issued March 14, 1989 and U.S. 4,833,125, Neer et al., issued May 23, 1989, all incorporated by reference herein.

The use of this invention is utilized until net skeletal mass is attained. Illustrative, but non-limiting, examples of the treatment regimens possible according to the methods of this invention are described herein: 1) a high potency phosphonate is orally administered at a level of about 0.005 mgP/kg for three (3) days, followed by a seven (7) day rest period, followed by the oral administration of a high potency phosphonate at a level of 0.005 mgP/kg for seven (7) days, followed by a three (3) day rest period; 2) a high potency phosphonate is orally administered at a level of 0.1 mgP/kg for seven (7) days, followed by a seven (7) day rest period, followed by the oral administration of a high potency phosphonate at a level of 0.1 mgP/kg for one (1) day, followed by a seven (7) day rest period; 3) a high potency phosphonate is subcutaneously administered at a level of 0.00001 mgP/kg for seven (7) days, followed by a one (1) day rest period, followed by the subcutaneous administration of a high potency phosphonate at a level of 0.00001 mgP/kg for seven (7) days, followed by a seven (7) day rest period; 4) a high potency phosphonate is subcutaneously administered at a level of 0.00001 mg P/kg for fourteen (14) days, followed by a three (3) day rest period, followed by the subcutaneous administration of a high potency phosphonate at a level of 0.00001 mgP/kg for fourteen (14) days, followed by a three (3) day rest period, followed by the oral administration of a high potency phosphonate at a level of 0.1 mgP/kg for fourteen (14) days; 5) a high potency phosphonate is subcutaneously administered at a level of 0.00001 mgP/kg for thirty (30) days followed by a one day (1) rest period, followed by the oral administration of a high potency phosphonate at a level of 0.01 mgP/kg for sixty (60) days, followed by a sixty (60) day rest period, followed by the oral administration of a high potency phosphonate at a level of 0.005 mgP/kg for sixty (60) days, followed by a thirty (30) day rest period; 6) a high potency phosphonate is orally administered at a level of 0.005 mgP/kg for three (3) days, followed by a seven (7) day rest period, followed by the oral administration of a high potency phosphonate at a level of 0.005 mgP/kg for three (3) days, followed by a seven (7) day rest period; 7) a high potency phosphonate is orally administered at a level of 0.001 mgP/kg for three hundred and sixty-five (365) days, the patient is then monitored for maintenance of skeletal mass; 8) a high potency phosphonate is orally administered at a level of 0.008 mgP/kg for fourteen (14) days, followed by a seven (7) day rest period, followed by the oral administration of a high potency phosphonate at a level of 0.004 mgP/kg for thirty (30) days, followed by a fourteen (14) days rest period, followed by the oral administration of a high potency phosphonate at a level of about 0.002 mgP/kg for sixty (60) days; 9) a high potency phosphonate is subcutaneously administered at a level of 0.00005 mgP/kg for sixty (60) days, followed by a thirty (30) day rest period, followed by the subcutaneous administration of a high potency phosphonate at a level of about 0.00001 mgP/kg for sixty (60) days, followed by a thirty (30) day rest period; 10) a high potency phosphonate is orally administered at a level of 0.005 mgP/kg for seventy (70) days, followed by a thirty (30) day rest period, followed by the oral administration of a high potency phosphonate at a level of 0.001 mgP/kg for ninety (90) days, followed by a seven (7) day rest period, followed by the oral administration of a high potency phosphonate at a level of 0.001 mgP/kg for seventy (70) days; 11) a high potency phosphonate is subcutaneously administered at a level of 0.0001 mgP/kg for one (1) day, followed by a one (1) day rest period; followed by the oral administration of a high potency phosphonate at a level of about 0.005 mgP/kg for one (1) day, followed by a one (1) day rest period; 12) a high potency phosphonate is orally administered at a level of 0.1 mgP/kg for one (1) day, followed by a fourteen (14) day rest period, followed by the oral administration of a high potency phosphonate at a level of 0.1 mgP/kg for fourteen (14) days, followed by a fourteen (14) day rest period, followed by the oral administration of a high potency phosphonate at a level of 0.001 mgP/kg for thirty (30) days; 13) a high potency phosphonate is subcutaneously administered at a level of 0.00001 mgP/kg for sixty (60) days, followed by a thirty (30) day rest period, followed by the subcutaneous administration of a high potency phosphonate at a level of 0.00001 mgP/kg for ninety (90) days, followed by a thirty (30) day rest period, followed by the oral administration of a high

potency phosphonate at a level of 0.001 mgP/kg for one hundred and eighty (180) days, followed by a thirty (30) day rest period.

Additionally, the following non-limiting clinical examples illustrate the compositions, processes and uses of the present invention.

EXAMPLE 1

A 62 kg postmenopausal Caucasian female, age 65 years old has experienced considerable bone loss since menopause and X-rays now reveal that she has two vertebral crush fractures which have caused her some pain. A 0.05 mg P/kg daily oral dose of 2-(3-pyridinyl)-1-hydroxyethane bisphosphonate is prescribed and administered for fourteen (14) days, followed by a seven (7) day rest period, followed by another fourteen (14) day 2-(3-pyridinyl)-1-hydroxyethane bisphosphonate administration period, followed by a seven (7) day rest period. At the end of one year, densitometry measurements indicate that the patient has 3% greater bone mass and the pain is gone. A 0.05 mgP/kg daily oral dose of 2-(3-pyridinyl)-1-hydroxyethane bisphosphonate is prescribed and administered for fourteen (14) days, followed by a fourteen (14) day rest period, followed by another fourteen (14) day 2-(3-pyridinyl)-1-hydroxyethane bisphosphonate administration period, followed by a fourteen (day) rest period. At the end of this one-year dosing period, bone mass is 7% greater than when therapy started and the patient has noticed greater mobility and increased quality of life. Due to these favorable results, the oral dosage regimen is continued at a level of 0.01 mgP/kg per day and annual bone densitometry measurements are scheduled.

EXAMPLE 2

A 64 kg Caucasian male, age 78 years old has over the last few years experienced reduced mobility and frequent back pain. Densitometry measurements reveal that the bone density in his spine and hip are well below normal and approaching the fracture threshold. X-rays of the spine show evidence of microfractures but no overt crushing. A cyclic therapy of 3-(2-hydroxy-2,2-diphosphonoethyl)-1-methyl pyridinium iodide disodium salt is prescribed. A 0.05 mg P/kg oral dose is given daily for 7 days followed by 21 days of free period. This cycle is repeated over a two year period, after which densitometry measurements indicate that the patient's bone density has increased by 5%. Furthermore, X-rays reveal that the microfractures have healed and the patient is free of back pain. At this point in time, therapy is discontinued but annual densitometry measurements are scheduled to monitor the patient's bone density.

EXAMPLE 3

A 45 year old Caucasian female entering menopause was found by bone densitometry measurements to have a vertebral bone density about 25% less than what would be expected. Without treatment, osteoporotic fractures would almost surely begin within five to ten years. Oral phosphonate therapy with 3-(2,2-diphosphonoethyl)-1-methyl pyridinium chloride was first prescribed at the well accepted antiresorptive dose level of 1.0 mg P/kg per day by mouth, but this was poorly tolerated due to esophageal and gastric irritation. The dose was then reduced to a subantiresorptive dose of 0.075 mg P/kg orally per day for a period of thirty (30) days, followed by a thirty (30) day rest period and was well tolerated. This particular cycle was continued for five years. Densitometry measurements during the five year period demonstrated a steadily increasing bone density. At the end of five years, the subject's bone density was found to be increased by 31% and therapy was discontinued with no further follow-up.

EXAMPLE 4

An 80 year old Black female was diagnosed by ultrasound densitometry as having very low bone mass, about 20% below the fracture threshold. X-rays of the spine revealed numerous minor vertebral fractures characteristic of senile osteoporosis. The patient's balance and mobility had deteriorated considerably during the last few years and because of her low bone density she was considered to be at risk for a hip fracture if she were to fall. Therapy with 3-(2,2-diphosphonoethyl)-1-(2-mercaptoethyl) pyridinium chloride was instituted. She was given 3-(2,2-diphosphonoethyl)-1-(2-mercaptoethyl) pyridinium chloride in an 84 day cycle, receiving 0.00005 mg P/kg subcutaneously per day for the first 14 days, followed by a 70 day rest period. A 1000 mg calcium supplement was administered during this seventy (70) day period. This cyclic therapy continued for a period of three years. At that time, the patient's bone density had increased by 12% and a spinal X-ray revealed that the vertebrae had healed adequately and no new fractures were evident. In the third year the patient fell while climbing stairs but did not fracture any bones. Therapy was continued and annual ultrasound densitometry measurements were scheduled to monitor the patient's bone density.

EXAMPLE 5

A 35 year old Hispanic male had a history of asthma which required glucocorticoid therapy for adequate control. Over several years, his bone mass had decreased steadily, as assessed by ultrasound densitometry measurements, and was now low enough to put him at risk of osteoporotic fractures. Therapy with 3-(N-pentyl-N-methyl)-amino-1-hydroxypropane-1,1-bisphosphonic acid was prescribed at a dose level of 0.001 mg P/kg per day orally and this was continued for a ten year period. During that time, ultrasound measurements were taken regularly and demonstrated a steady increase in bone mass of about 2% per year. At the end of the ten year period, the patient (now 45 years old) found that his asthma had improved and that the glucocorticoid therapy was no longer needed. Bone mass was also judged to be within acceptable limits and the therapy was discontinued.

EXAMPLE 6

A 72 year old Japanese female had been treated for several years with steroids in an attempt to control her rheumatoid arthritis. The patient's reduction in bone (almost 30% below peak menopausal bone density) was of sufficient concern as to call for immediate therapy. The subject was treated with N-(4-hydroxy-4,4-diphosphonobutyl)-N,N,N-trimethylammonium iodide at a level of 0.0001 mgP per day subcutaneously for 60 days. Following a 14-day period without drug administration, she was then placed on an oral maintenance dose of 0.05 mg P/kg per day for fourteen (14) days followed by a seven (7) day rest period. This oral dosing maintenance regimen is repeated for a period of a 1 year. Bone densitometry measurements during that period demonstrated that her bone density had stabilized and at the end of 1 year was 2% higher than at the time treatment was started.

EXAMPLE 7

A 69 year old Oriental female complained to her family doctor about back pain and was referred to a radiologist for X-rays and bone densitometry measurements. These assessments revealed that her bone density was well below peak menopausal bone mass and that she had already sustained several vertebral deformations. She was referred to an endocrinologist, who prescribed a treatment regimen of N-cycloheptylaminomethane bisphosphonate at a subcutaneous level of 0.00003 mgP/kg per day for thirty days. At the end of that 30 day period, she was prescribed an oral dosage regimen of 0.003 mgP/kg for 365 days. Subsequent densitometry measurements indicated that her bone density had improved by 5%. Oral therapy was continued at a level of 0.001 mgP/kg per day with annual monitoring of bone density.

EXAMPLE 8

A 76 year old Caucasian woman weighing 55 kg and with a family history of osteoporosis was admitted to the hospital with a compound fracture of the wrist, this injury resulting from a fall in her home. Following surgery for repair of the wrist fracture, her orthopedic surgeon ordered bone densitometry measurements and discovered that her bone density was very low. In order to augment her bone density, the surgeon prescribed 4-amino-1-hydroxy butane-1,1-bisphosphonic acid by subcutaneous administration. For 1 month she was given 0.00025 mg P/kg per day by subcutaneous injection. This was followed by a daily oral maintenance dose of 0.025 mg P/kg for 30 months. During her therapy, bone density was monitored every 6 months using an ultrasound densitometer. Her bone density first stabilized and then steadily increased and had increased by a total of 10% after the 30 months of therapy.

## Claims

1. The use of a high potency phosphonate in the manufacture of a medicament to be used for increasing bone mass in a human or other mammal subject afflicted with osteoporosis, comprising a thirty (30) day treatment period, comprised of a high potency phosphonate compound administration regimen wherein

   (a) said high potency phosphonate administration regimen comprises the systemic administration to said subject of a high potency phosphonate compound at a level of from 0.00001 mgP/kg to 0.1 mgP/kg per day that said high potency phosphonate compound is administered, provided that said level of the high potency phosphonate compound is administered at least 1 day of every said thirty (30) day treatment period; and wherein
   (b) said thirty (30) day treatment period may be followed by a rest period of at least one day.

2. The use of a high potency phosphonate, according to Claim 1, wherein the medicament is to be used before a significant loss of net skeletal mass as occurred in said subject.

3. The use of a high potency phosphonate, according to Claim 1, wherein said high potency phosphonate is a bisphosphonic acid, a phosphonoalkylphosphinate, a phosphonosulfonate or a phosphonocarboxylate, preferably a bisphosphonic acid and the pharmaceutically-acceptable salt or ester thereof.

4. The use of a high potency phosphonate, according to Claim 1, wherein said high potency phosphonate compound is of the formula:

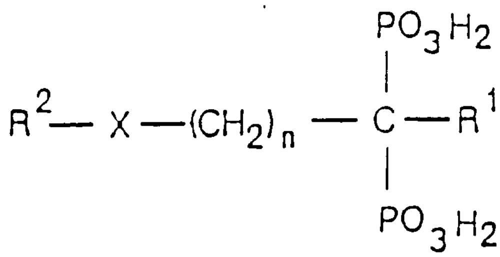

wherein: n is an integer from 0 to 7; $R^1$ is hydrogen, chloro, amino, or hydroxy; X is -NH-, oxygen, or a single bond; $R^2$ is a 5- to 7-membered heterocycle having from 1 to 3 heteroatoms, 5-to 7- membered quaternary nitrogen containing heterocycle; and their pharmaceutically-acceptable salts and esters.

5. The use of a high potency phosphonate, according to Claim 1, wherein said high potency phosphonate compound is selected from the group consisting of: 4-amino-1-hydroxybutane-1,1-bisphosphonic acid; 2-(3-pyridyl)-1-hydroxyethane-1,1-bisphosphonic acid; 2-(N-imidaxoyl)-1-hydroxyethane-1,1-bisphosphonic acid; 3-(N-pentyl-N-methyl)-amino-1-hydroxypropane-1,1-bisphosphonic acid; 3-(N-pyrollidino)-1-hydroxypropane-1,1-bisphosphonic acid; 2-(6-pyrolopyridine)-1-hydroxyethane-1,1-bisphosphonic acid; 2-(2-pyridyl)-1-hydroxy-ethane-1,1-bisphosphonic acid; N-cycloheptylaminomethane bisphosphonic acid; 3-(N,N-dimethyl)amino-1-hydroxypropane-1,1-bisphosphonic acid and pharmaceutically-acceptable salts and esters thereof.

6. The use of a high potency phosphonate, according to Claim 5, wherein said high potency phosphonate compound is 2-(3-pyridyl)-1-hydroxyethane-1,1-bisphosphonic acid, or a pharmaceutically acceptable salt or ester thereof.

7. The use of a high potency phosphonate, according to Claim 1, wherein said high potency phosphonate compound is selected from the group consisting of: 2-(2-hydroxy-2,2-diphosphonoethyl)-1,1-dimethylpiperidinium iodide salt; 3-(2-hydroxy-2,2-diphosphonoethyl)-1-methylpyridinium iodide; 3-(2-hydroxy-2,2-diphosphonoethyl)-1-methylpyridinium hydroxide; 3-(2,2-diphosphonoethyl)-1-ethylpyridinium chloride; 3-(2,2-diphosphonoethyl)-1-(2-mercaptoethyl)pyridinium chloride; 2-(2-hydroxy-2,2-diphosphonoethyl)-1-methylpyridinium hydroxide; 3-(3-hydroxy-3,3-diphosphonopropyl)-1-methylpyridinium hydroxide; 3-(2,2-diphosphono-2-hydroxyethyl)-1,1-dimethylpiperidinium iodide salt; 3-(2,2-diphosphonoethyl)-1-heptylpyridinium chloride; 3-(2,2-diphosphonoethyl)-1-methylpyridinium chloride; 3-(2,2-phosphonomethylphosphinoethyl)-1-methylpyridinium iodide; 3-(2-phosphono-2-sulfonoethyl)-1-methylpyridinium chloride; 3-(2-carboxy-2-phosphonoethyl)-1-methylpyridinium chloride; 2-diphosphonomethyl-1,1-dimethylpiperidinium chloride; 3-diphosphonomethyl-1,1-dimethylpiperidinium chloride; 4-diphosphonomethyl-1,1-dimethylpiperidinium chloride; 2-(2,2-diphosphonoethyl)-1,1-dimethylpiperidinium chloride; 3-(2,2-diphosphonoethyl)-1,1-dimethylpiperidinium chloride; 4-(2,2-diphosphonoethyl)-1,1-dimethylpiperidinium chloride; 2-(2,2-diphosphonoethyl)-1-methyl-1-(2-mercaptoethyl)piperidinium chloride; 3-(2,2-diphosphonoethyl)-1-methyl-1-(2-mercaptoethyl)piperidinium chloride; 4-(2,2-diphosphonoethyl)-1-methyl-1-(2-mercaptoethyl)piperidinium chloride; 2-[2,2-diphosphono-1-(2-mercaptoethyl)ethyl]-1,1-dimethylpiperidinium chloride; 3-[2,2-diphosphono-1-(3-mercaptopropyl)ethyl]-1,1-dimethylpiperidinium chloride; 4-[2,2-diphosphono-1-(2-acetylthioethyl)ethyl]-1,1-dimethylpiperidinium chloride; 2-(2,2-diphosphono-2-hydroxyethyl)-1,1-dimethylpiperidinium chloride; 3-(2,2-diphosphono-2-hydroxyethyl)-1,1-dimethylpiperidinium chloride; 4-(2,2-diphosphono-2-hydroxyethyl) -1,1-dimethylpiperidinium chloride; 2-(2,2-diphosphono-2-hydroxy ethyl)-1,1,3-trimethylpiperidinium chloride; 2-(2,2-diphosphono-2-hydroxyethyl)-1,1,5-trimethylpiperidinium chloride; 2-(2,2-diphosphonoethyl)-1,1,3-trimethylpiperidinium chloride; 2-(2,2-diphosphonoethyl)-1,1,5-trimethylpiperidinium chloride; 2-(3,3-diphosphonopropyl)-1,1-dimethylpiperidinium chloride; 3-(3,3-diphosphonopropyl)-1,1-dimethylpiperidinium chloride; 4-(3,3-diphosphonopropyl)-1,1-dimethylpiperidinium chloride; 2-(3,3-diphosphono-3-hydroxypropyl)-1,1-dimethylpiperidinium chloride; 3-(3,3-diphosphono-3-hydroxypropyl)-1,1-dimethylpiperidinium chloride; 4-(3,3-diphosphono-3-hydroxypropyl)-1,1-dimethylpiperidinium chloride; 2-(2,2-diphosphonopropyl)-1,1-dimethylpiperidinium chloride; 3-(2,2-diphosphonopropyl)-1,1-dimethylpiperidinium chloride; 4-(2,2-diphosphonopropyl)-1,1-dimethylpiperidinium chloride; 2-(2,2-diphosphono-2-aminoethyl)-1,1-dimethylpiperidinium chloride; 3-(2,2-diphosphono-2-aminoethyl)-1,1-dimethylpiperidinium chloride; 4-(2,2-diphosphono-2-aminoethyl)-1,1-dimethylpiperidinium chloride; 2-(2,2-diphosphono-2-aminoethyl)-1,1,3-trimethylpiperidinium chloride; 2-(2,2-diphosphono-2-aminoethyl)-1,1,3-trimethylpiperidinium chloride; 3-(2,2-diphosphono-2-aminoethyl)-1,1,5-trimethylpiperidinium chloride; 2-(2,2-diphosphono-2-(methylamino)ethyl)-1,1,-dimethylpiperidinium chloride; 2-(4,4-diphosphono-4-hydroxybutyl) -1,1,3-trimethylpiperidinium chloride; 2-(4,4-diphosphono-4-hydroxybutyl)-1,1-dimethylpiperidinium chloride; 2-(2,2-diphosphono-2-hydroxyethyl)-3-carboxy-1,1-dimethylpiperidinium chloride; 2-(2,2-diphosphono-2-hydroxyethyl)-5-carboxy-1,1-dimethylpiperidinium chloride; 2-(2,2-diphosphonoethyl)-1-methylpyrimidinium chloride; 4-(2,2-diphosphonoethyl)-1-methylpyrimidinium chloride; 2-(2,2-diphosphono-2-hydroxyethyl)-1-methylpyrimidinium chloride; 4-(2,2-diphosphono-2-hydroxyethyl) -1-methylpyrimidinium chloride; 2-(3,3-diphosphonopropyl)-1-methylpyrimidinium chloride; 4-(3,3-diphosphonopropyl)-1-methylpyrimidinium chloride; 2-(3,3-diphosphono-1-hydroxypropyl)-1-methylpyrimidinium chloride; 4-(3,3-diphosphono-1-hydroxypropyl)-1-methylpyrimidinium chloride; 2-(2,2-diphosphono-2-aminoethyl)-1-methylpyrimidinium chloride; 3-[(diphosphonomethyl)oxo]-1,1-dimethylpiperidinium chloride; 4-[(diphosphonomethyl)oxo]-1,1-dimethylpiperidinium chloride; 3-[(2,2-diphosphonoethyl)oxo]-1,1-dimethylpiperidinium chloride; 4-[(2,2-diphosphonoethyl)oxo]-1,1-dimethylpiperidinium chloride; 3-[(diphosphonomethyl)thio]-1,1-dimethylpiperidinium chloride; 4-[(diphosphonomethyl)thio]-1,1-dimethylpiperidinium chloride; 3-(2-hydroxy-2,2-diphosphonoethyl)-1-methylpyridinium iodide; 3-(2-hydroxy-2,2-diphosphonoethyl)-1-methylpyridinium hydroxide; 3-(2,2-diphosphonoethyl)-1-(2-mercaptoethyl)pyridinium chloride; 2-(2-hydroxy-2,2-diphosphonoethyl)-1,1-dimethylpiperidinium iodide salt; 3-(2,2-diphosphono-2-hydroxyethyl)-1,1-dimethylpiperidinium iodide salt; 3-(2,2-diphosphonoethyl)-1-heptylpyridinium chloride; 3-(2,2-diphosphonoethyl)-1-methylpyridinium chloride; 2-(2,2-diphosphonoethyl)-1,1-dimethylpiperidinium chloride; 3-(2,2-diphosphonoethyl)-1,1-dimethylpiperidinium chloride; 4-(2,2-diphosphonoethyl)-1,1-dimethylpiperidinium chloride; 2-(2,2-diphosphono-2-hydroxyethyl)-1,1-dimethylpiperidinium chloride; 3-(2,2-diphosphono-2-hydroxyethyl)-1,1-dimethylpiperidinium chloride; 4-(2,2-diphosphono-2-hydroxyethyl)-1,1-dimethylpiperidinium chloride; 2-(2,2-diphosphono-2-hydroxyethyl)-1,1,3-trimethylpiperidinium chloride; 2-(2,2-diphosphono-2-hydroxyethyl)-1,1,5-trimethylpiperidinium chloride; 2-[2,2-diphosphono-1-(2-mercaptoethyl)ethyl]-1,1-dimethylpiperidinium chloride; 3-[2,2-diphosphono-1-(3-mercaptopropyl)ethyl]-1,1-dimethylpiperidinium chloride; 2-(2,2-diphosphonoethyl)-1-methyl-1-(2-mercaptoethyl)piperidinium chloride; 3-(2,2-diphosphonoethyl)-1-methyl-1-(2-mercaptoethyl)piperidinium chloride; 4-(2,2-diphosphonoethyl)-1-methyl-1-(2-mercaptoethyl) piperidinium chloride; 3-(2-hydroxy-2,2-diphosphonoethyl)-1-methylpyridinium iodide; 3-(2-hydroxy-2,2-diphosphonoethyl)-1-methylpyridinium hydroxide; 3-(2,2-diphosphonoethyl)-1-(2-mercaptoethyl)pyridinium chloride; 2-[2,2-diphosphono-1-(2-mercaptoethyl)ethyl]-1,1-dimethylpiperidinium chloride; 3-[2,2-diphosphono-1-(3-mercaptopropyl)ethyl]-1,1-dimethylpiperidinium chloride; 2-(2,2-diphosphonoethyl)-1-methyl-1-(2-mercaptoethyl) piperidinium chloride; 3-(2,2-diphosphonoethyl)-1-methyl-1-(2-mercaptoethyl)piperidinium chloride; and the pharmaceutically-acceptable salts and esters thereof.

8. The use of a high potency phosphonate, according to Claim 1, wherein said high potency phosphonate compound is selected from the group consisting of N-(4-hydroxy-4,4-diphosphonobutyl)-N,N,N-trimethyl ammonium iodide; N-(3-hydroxy-3,3-diphosphonopropyl)-N,N-dimethyl-N-pentyl ammonium iodide and the pharmaceutically-acceptable salts and esters thereof.

9. The use of a high potency phosphonate, according to Claim 1, wherein said high potency phosphonate compound is selected from the group consisting of [(5-[mercaptomethyl]-2-piperidinyl)methylene]bis[phosphonic acid; [(5-mercaptomethyl-3-piperidinyl]methylene]bis[phosphonic acid; [(5-mercapto-2-piperidinyl)methylene]bis[phosphonic acid; [(5-[4-mercaptobutyl]-2-piperidinyl)methylene]bis[phosphonic acid; [(5-mercapto-3-piperidinyl)methylene]bis[phosphonic acid; [(5-[5-mercaptopentyl]-3-piperidinyl)methylene]bis[phosphonic acid; [(5-[2-mercaptoethyl]-4-piperidinyl)methylene]bis[phosphonic acid; [(5-mercapto-4-piperidinyl)methylene]bis[phosphonic acid; [2-(5-mercapto-2-piperidinyl)ethylidene]bis[phosphonic acid]; [2-(5-[3-mercaptopropyl]-2-piperidinyl)ethylidene]bis[phosphonic acid]; [2-(5-mercapto-3-piperidinyl)ethylidene]bis[phosphonic acid]; [2-(5-mercapto-4-piperidinyl)ethylidene]bis[phosphonic acid]; [2-(5-[4-mercaptobutyl]-2-piperidinyl)ethylidene]bis[phosphonic acid]; [2-(5-mercaptomethyl-3-piperidinyl)ethylidene]bis[phosphonic acid]; [(2-[5-mercapto-2-piperidinyl]-1-hydroxy)ethylidene]bis[phosphonic acid]; [(2-[5-(3-mercaptopropyl)-2-piperidinyl]-1-hydroxy)ethylidene]bis[phosphonic acid] [(2-[5-mercapto-3-piperidinyl]-1-hydroxy)ethylidene]bis[phosphonic acid]; [(2-[5-(2-mercaptoethyl)3-piperidinyl]-1-hydroxy)ethylidene]-bis[phosphonic acid]; [(2-[5-mercapto-4-piperidinyl]-1-hydroxy)ethylidene]bis[phosphonic acid]; [(2-[5-mercaptomethyl-4-piperidinyl]-1-hydroxy)ethylidene]bis[phosphonic acid]; [(2-[5-mercaptomethyl-3-methyl-2-piperidinyl]-1-hydroxy)-ethylidene]bis[phosphonic acid]; [(2-[5-mercapto-3-methyl-2-piperidinyl]-1-hydroxy)ethylidene]bis[phosphonic acid]; [(2-[3-mercaptomethyl-5-methyl-2-piperidinyl]-1-hydroxy)-ethylidene]bis[phosphonic acid]; [2-(5-mercaptomethyl-3-methyl-2-piperidinyl)-ethylidene]bis[phosphonic acid]; [2-(3-mercaptomethyl-5-methyl-2-piperidinyl)ethylidene]bis[phosphonic acid]; [3-[5-(mercaptomethyl)-2-piperidinyl]propylidene]bis[phosphonic acid]; [3-[5-(mercaptomethyl)-3-piperidinyl]propylidene]bis[phosphonic acid]; [3-[5-(mercaptomethyl)-4-piperidinyl]propylidene]bis[phosphonic acid]; [3-[5-(mercaptomethyl)-2-piperidinyl]-1-hydroxypropylidene]bis[phosphonic acid]; [3-[5-mercapto-3-piperidinyl]-1-hydroxypropylidene]-bis[phosphonic acid]; [3-[5-(4-mercaptobutyl)-4-piperidinyl]-1-hydroxypropylidene]-bis[phosphonic acid]; [2-(3-mercaptomethyl-5-methyl-2-pyridinyl)ethylidene]-bis[phosphonic acid]; [2-(5-[3-mercaptopropyl]-2-methyl-2-piperidinyl)ethylidene]bis[phosphonic acid]; [(2-[5-(2-mercaptopropyl)-2-piperidinyl]-1-amino)ethylidene]bis[phosphonic acid]; [(2-[5-(3-mercaptopropyl)-3-piperidinyl]-1-amino)ethylidene]bis[phosphonic acid]; [2-(5-[3-mercaptopropyl]-4-piperidinyl)-1-aminoethylidene]bis[phosphonic acid]; [(2-[3-methyl-5-(3-mercaptopropyl)-2-piperidinyl]-1-hydroxy)ethylidene]bis[phosphonic acid]; [(2-[3-amino-5-(3-mercaptopropyl)-2-piperidinyl]-1-hydroxy)ethylidene]bis[phosphonic acid]; [2-[5-mercapto-2-(1,4-diazinyl)]ethylidene]bis[phosphonic acid]; [2-[5-(3-mercaptopropyl)-2-(1,4-diazinyl)]ethylidene]bis[phosphonic acid]; [2-[5-(3-mercaptopropyl)-2-(1,4-diazinyl)]-1-hydroxyethylidene]bis[phosphonic acid]; [2-[5-mercapto-2-(1,4-diazinyl)]-1-hydroxyethylidene]bis[phosphonic acid]; [2-[5-mercapto-2-(1,3-diazinyl)]ethylidene]bis[phosphonic acid] [2-[5-(3-mercaptopropyl)-2-(1,3-diazinyl)]ethylidene]bis[phosphonic acid]; [2-[5-(3-mercaptopropyl)-2-(1,3-diazinyl)]-1-hydroxyethylidene]bis[phosphonic acid]; [2-[5-mercapto-2-(1,3-diazinyl)]-1-hydroxyethylidene]bis[phosphonic acid]; [(5-[3-mercaptopropyl]-2-piperidinyl)aminomethylene]bis[phosphonic acid]; [(5-mercapto-2-piperidinyl)aminomethylene]bis[phosphonic acid]; [(5-[3-mercaptopropyl]-3-piperidinyl)aminomethylene]bis[phosphonic acid]; [(5-mercapto-3-piperidinyl)aminomethylene]bis[phosphonic acid]; [(5-mercapto-4-piperidinyl)aminomethylene]bis[phosphonic acid]; [(5-[3-mercaptopropyl]-4-piperidinyl)aminomethylene]bis[phosphonic acid]; [(5-mercapto-3-methyl-2-piperidinylidene)aminomethylene]bis[phosphonic acid]; [(5-[3-mercaptopropyl]-3-methyl-2-piperidinylidene)aminomethylene]bis[phosphonic acid]; [2-(5-mercapto-3-methyl-2-piperidinylidene)aminoethylene]bis[phosphonic acid]; [2-(5-[3-mercaptopropyl]-3-methyl-2-piperidinylidene)aminomethylene]bis[phosphonic acid]; [(5-mercapto-2-piperidinylidene)aminomethylene]bis[phosphonic acid]; [(5-[3-mercaptopropyl]-2-piperidinylidene)aminomethylene]bis[phosphonic acid]; [2-(5-mercapto-2-piperidinylidene)aminoethylene]bis[phosphonic acid] [(5-[3-mercaptopropyl]-2-piperidinylidene)aminomethylene]bis[phosphonic acid]; [(5-[3-mercaptopropyl]-2-[1,4-diazinylidene])aminomethylene]bis[phosphonic acid]; [(5-[3-mercaptopropyl]-2-[1,3-diazinylidene])aminomethylene]bis[phosphonic acid]; [(4-[3-mercaptopropyl]-2-[1,3,5-triazinylidene])aminomethylene]bis[phosphonic acid]; N-(2'-(1',3'-diazinylidene))-aminomethane diphosphonic acid; and the pharmaceutically-acceptable salts and esters thereof.

**10.** The use of a high potency phosphonate according to Claim 1, wherein said high potency phosphonate is a phosphonosulfonate, a phosphonocarboxylate, or a phosphonoalkylphosphinate and the pharmaceutically-acceptable salt or ester thereof.

## Patentansprüche

**1.** Verwendung eines hochwirksaman Phosphonats zur Herstellung eines Arzneimittels, welches für die Erhöhung der Knochenmasse beim Menschen oder einem anderen Säugetierpatienten, welcher unter Osteoporose leidet, verwendet werden soll, umfassend eine dreißigtägige (30-tägige) Behandlungsdauer, welche ein Regime zur Verabreichung einer hochwirksamen Phosphonatverbindung beinhaltet, wobei

(a) das genannte Regime zur Verabreichung eines hochwirksamen Phosphonats die systemische Verabreichung einer hochwirksamen Phosphonatverbindung an den genannten Patienten in einer Menge von 0,00001 mgP/kg bis 0,1 mgP/kg pro Tag, an welchem die hochwirksame Phosphonatverbindung verabreicht wird, umfaßt, unter der Voraussetzung, daß die genannte Menge der hochwirksamen Phosphonatverbindung mindestens 1 mal täglich in der genannten dreißigtägigen (30-tägigen) Behandlungsdauer verabreicht wird; und worin

(b) die genannte dreißigtägige (30-tägige) Behandlungsdauer von einer Ruheperiode von mindestens einem Tag gefolgt sein kann.

**2.** Verwendung eines hochwirksamen Phosphonats nach Anspruch 1, wobei das Arzneimittel verwendet wird, bevor ein beträchtlicher Verlust an Nettoskelettmasse beim genannten Patienten aufgetreten ist.

**3.** Verwendung eines hochwirksamen Phosphonats nach Anspruch 1, wobei das genannte hochwirksame Phosphonat eine Bisphosphonsäure, ein Phosphonoalkylphosphinat, ein Phosphonosulfonat oder ein Phosphonocarboxylat, vorzugsweise eine Bisphosphonsäure, und ein pharmazeutisch annehmbares Salz oder ein pharmazeutisch annehmbarer Ester hievon ist.

4. Verwendung eines hochwirksamen Phosphonats nach Anspruch 1, wobei die genannte hochwirksame Phosphonatverbindung die Formel

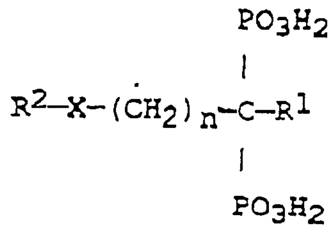

besitzt, worin n für eine ganze Zahl von 0 bis 7 steht; $R^1$ Wasserstoff, Chlor, Amino oder Hydroxy bedeutet; X für -NH-, Sauerstoff oder eine Einfachbindung steht; $R^2$ einen 5- bis 7-gliedrigen Heterocyclus mit 1 bis 3 Heteroatomen; einen 5- bis 7-gliedrigen, einen quaternären Stickstoff enthaltenden Heterocyclus darstellt; und ein pharmazeutisch annehmbares Salz und ein pharmazeutisch annehmbarer Ester ist.

5. Verwendung eines hochwirksamen Phosphonats nach Anspruch 1, wobei die genannte hochwirksame Phosphonatverbindung von der Gruppe ausgewählt ist, welche aus 4-Amino-1-hydroxybutan-1,1-bisphosphonsäure; 2-(3-Pyridyl)-1-hydroxyethan-1,1-bisphosphonsäure; 2-(N-Imidazoyl)-1-hydroxyethan-1,1-bisphosphonsäure; 3-(N-Pentyl-N-methyl)-amino-1-hydroxypropan-1,1-bisphosphonsäure; 3-(N-Pyrollidino)-1-hydroxypropan-1,1-bisphosphonsäure; 2-(6-Pyrolopyridin)-1-hydroxyethan-1,1-bisphosphonsäure; 2-(2-Pyridyl)-1-hydroxyethan-1,1-bisphosphonsäure; N-Cycloheptylaminomethanbisphosphonsäure; 3-(N,N-Dimethyl)amino-1-hydroxypropan-1,1-bisphosphonsäure und den pharmazeutisch annehmbaren salzen und Estern hievon besteht.

6. Verwendung eines hochwirksamen Phosphonats nach Anspruch 5, wobei die genannte hochwirksame Phosphonatverbindung 2-(3-Pyridyl)-1-hydroxyethan-1,1-bisphosphonsäure oder ein pharmazeutisch annehmbares Salz oder ein Ester hievon ist.

7. Verwendung eines hochwirksamen Phosphonats nach Anspruch 1, wobei die genannte hochwirksame Phosphonatverbindung von der Gruppe ausgewählt ist, welche aus 2-(2-Hydroxy-2,2-diphosphonoethyl)-1,1-dimethylpiperidiniumiodidsalz; 3-(2-Hydroxy-2,2-diphosphonoethyl)-1-methylpyridiniumiodid; 3-(2-Hydroxy-2,2-diphosphonoethyl)-1-methylpyridiniumhydroxid; 3-(2,2-Diphosphonoethyl)-1-ethylpyridiniumchlorid; 3-(2,2-Diphosphonoethyl)-1-(2-mercaptoethyl)pyridiniumchlorid; 2-(2-Hydroxy-2,2-diphosphonoethyl)-1-methylpyridiniumhydroxid; 3-(3-Hydroxy-3,3-diphosphonopropyl)-1-methylpyridiniumhydroxid; 3-(2,2-Diphosphono-2-hydroxyethyl)-1,1-dimethylpiperidiniumiodidsalz; 3-(2,2-Diphosphonoethyl)-1-heptylpyridiniumchlorid; 3-(2,2-Diphosphonoethyl)-1-methylpyridiniumchlorid; 3-(2,2-Phosphonomethylphosphinoethyl)-1-methylpyridiniumiodid; 3-(2-Phosphono-2-sulfonoethyl)-1-methylpyridiniumchlorid; 3-(2-Carboxy-2-phosphonoethyl)-1-methylpyridiniumchlorid; 2-Diphosphonomethyl-1,1-dimethylpiperidiniumchlorid; 3-Diphosphonomethyl-1,1-dimethylpiperidiniumchlorid; 4-Diphosphonomethyl-1,1-dimethylpiperidiniumchlorid; 2-(2,2-Diphosphonoethyl)-1,1-dimethylpiperidiniumchlorid; 3-(2,2-Diphosphonoethyl)-1,1-dimethylpiperidiniumchlorid; 4-(2,2-Diphosphonoethyl)-1,1-dimethylpiperidiniumchlorid; 2-(2,2-Diphosphonoethyl)-1-methyl-1-(2-mercaptoethyl)piperidiniumchlorid; 3-(2,2-Diphosphonoethyl)-1-methyl-1-(2-mercaptoethyl)piperidiniumchlorid; 4-(2,2-Diphosphonoethyl)-1-methyl-1-(2-mercaptoethyl)piperidiniumchlorid; 2-[2,2-Diphosphono-1-(2-mercaptoethyl)ethyl]-1,1-dimethylpiperidiniumchlorid; 3-[2,2-Diphosphono-1-(3-mercaptopropyl)ethyl]-1,1-dimethylpiperidiniumchlorid; 4-[2,2-Diphosphono-1-(2-acetylthioethyl)ethyl]-1,1-dimethylpiperidiniumchlorid; 2-(2,2-Diphosphono-2-hydroxyethyl)-1,1-dimethylpiperidiniumchlorid; 3-(2,2-Diphosphono-2-hydroxyethyl)-1,1-dimethylpiperidiniumchlorid; 4-(2,2-Diphosphono-2-hydroxyethyl)-1,1-dimethylpiperidiniumchlorid; 2-(2,2-Diphosphono-2-hydroxyethyl)-1,1,3-trimethylpiperidiniumchlorid; 2-(2,2-Diphosphono-2-hydroxyethyl)-1,1,5-trimethylpiperidiniumchlorid; 2-(2,2-Diphosphonoethyl)-1,1,3-trimethylpiperidiniumchlorid; 2-(2,2-Diphosphonoethyl)-1,1,5-trimethylpiperidiniumchlorid; 2-(3,3-Diphosphonopropyl)-1,1-dimethylpiperidiniumchlorid; 3-(3,3-Diphosphonopropyl)-1,1-dimethylpiperidiniumchlorid; 4-(3,3-Diphosphonopropyl)-1,1-dimethylpiperidiniumchlorid; 2-(3,3-Diphosphono-3-hydroxpropyl)-1,1-dimethylpiperidiniumchlorid; 3-(3,3-Diphosphono-3-hydroxypropyl)-1,1-dimethylpiperidiniumchlorid; 4-(3,3-Diphosphono-3-hydroxypropyl)-1,1-dimethylpiperidiniumchlorid; 2-(2,2-Diphosphonopropyl)-1,1-dimethylpiperidiniumchlorid; 3-(2,2-Diphosphonopropyl)-1,1-dimethylpiperidiniumchlorid; 4-(2,2-Diphosphonopropyl)-1,1-dimethylpiperidiniumchlorid; 2-(2,2-Diphosphono-2-aminoethyl)-1,1-dimethylpiperidiniumchlorid; 3-(2,2-Diphosphono-2-aminoethyl)-1,1-dimethylpiperidiniumchlorid; 4-(2,2-Diphosphono-2-aminoethyl)-1,1-dimethylpiperidiniumchlorid; 2-(2,2-Diphosphono-2-aminoethyl)-1,1,3-trimethylpiperidiniumchlorid; 2-(2,2-Diphosphono-2-aminoethyl)-1,1,3-trimethylpiperidiniumchlorid; 3-(2,2-Diphosphono-2-aminoethyl)-1,1,5-trimethylpiperidiniumchlorid; 2-(2,2-Diphosphono-2-(methylamino)ethyl)-1,1,-dimethylpiperidiniumchlorid; 2-(4,4-Diphosphono-4-hydroxybutyl)-1,1,3-trimethylpiperidiniumchlorid; 2-(4,4-Diphosphono-4-hydroxybutyl)-1,1-dimethylpiperidiniumchlorid; 2-(2,2-Diphosphono-2-hydroxyethyl)-3-carboxy-1,1-dimethylpiperidiniumchlorid; 2-(2,2-Diphosphono-2-hydroxyethyl)-5-carboxy-1,1-dimethylpiperidiniumchlorid; 2-(2,2-Diphosphonoethyl)-1-methylpyrimidiniumchlorid; 4-(2,2-Diphosphonoethyl)-1-methylpyrimidiniumchlorid; 2-(2,2-Diphosphono-2-hydroxyethyl)-1-methylpyrimidiniumchlorid; 4-(2,2-Diphosphono-2-hydroxyethyl)-1-methylpyrimidiniumchlorid; 2-(3,3-Diphosphonopropyl)-1-methylpyrimidiniumchlorid; 4-(3,3-Diphosphonopropyl)-1-methylpyrimidiniumchlorid; 2-(3,3-Diphosphono-1-hydroxypropyl)-1-methylpyrimidiniumchlorid; 4-(3,3-Diphosphono-1-hydroxypropyl)-1-methylpyrimidiniumchlorid; 2-(2,2-Diphosphono-2-aminoethyl)-1-methylpyrimidiniumchlorid; 3-[(Diphosphonomethyl)oxo]-1,1-dimethylpiperidiniumchlorid; 4-[(Diphosphonomethyl)oxo]-1,1-dimethylpiperidiniumchlorid; 3-[(2,2-Diphosphonoethyl)oxo]-1,1-dimethylpiperidiniumchlorid; 4-[(2,2-Diphosphonoethyl)oxo]-1,1-dimethylpiperidiniumchlorid; 3-[(Diphosphonomethyl)thio]-1,1-dimethylpiperidiniumchlorid; 4-[(Diphosphonomethyl)thio]-1,1-dimethylpiperidiniumchlorid: 3-(2-Hydroxy-2,2-diphosphonoethyl)-1-methylpyridiniumiodid; 3-(2-Hydroxy-2,2-diphosphonoethyl)-1-methylpyridiniumhydroxid; 3-(2,2-Diphosphonoethyl)-1-(2-mercaptoethyl)pyridiniumchlorid; 2-(2-Hydroxy-2,2-diphosphonoethyl)-1,1-dimethylpiperidiniumiodidsalz; 3-(2,2-Diphosphono-2-hydroxyethyl)-1,1-dimethylpiperidiniumiodidsalz; 3-(2,2-Diphosphonoethyl)-1-heptylpyridiniumchlorid; 3-(2,2-Diphosphonoethyl)-1-methylpyridiniumchlorid; 2-(2,2-Diphosphonoethyl)-1,1-dimethylpiperidiniumchlorid; 3-(2,2-Diphosphonoethyl)-1,1-dimethylpiperidiniumchlorid; 4-(2,2-Diphosphonoethyl)-1,1-dimethylpiperidiniumchlorid; 2-(2,2-Diphosphono-2-hydroxyethyl)-1,1-dimethylpiperidiniumchlorid; 3-(2,2-Diphosphono-2-hydroxyethyl)-1,1-dimethylpiperidiniumchlorid; 4-(2,2-Disphosphono-2-hydroxyethyl)-1,1-dimethylpiperidiniumchlorid; 2-(2,2-Diphosphono-2-hydroxyethyl)-1,1,3-trimethylpiperidiniumchlorid; 2-(2,2-Diphosphono-2-hydroxyethyl)-1,1,5-trimethylpiperidiniumchlorid; 2-[2,2-Diphosphono-1-(2-mercaptoethyl)ethyl]-1,1-dimethylpiperidiniumchlorid; 3-[2,2-Diphosphono-1-(3-mercaptopropyl)ethyl]-1,1-dimethylpiperidiniumchlorid; 2-(2,2-Diphosphonoethyl)-1-methyl-1-(2-mercaptoethyl)piperidiniumchlorid; 3-(2,2-Diphosphonoethyl)-1-methyl-1-(2-mercaptoethyl)piperidiniumchlorid; 4-(2,2-Diphosphonoethyl)-1-methyl-1-(2-mercaptoethyl)piperidiniumchlorid; 3-(2-Hydroxy-2,2-diphosphonoethyl)-1-methylpyridiniumiodid; 3-(2-Hydroxy-2,2-diphosphonoethyl)-1-methylpyridiniumhydroxid; 3-(2,2-Diphosphonoethyl)-1-(2-mercaptoethyl)pyridiniumchlorid; 2-[2,2-Diphosphono-1-(2-mercaptoethyl)ethyl]-1,1-dimethylpiperidinium-chlorid; 3-[2,2-Diphosphono-1-(3-mercaptopropyl)ethyl]-1,1-dimethylpiperidiniumchlorid; 2-(2,2-Diphosphonoethyl)-1-methyl-1-(2-mercaptoethyl)piperidiniumchlorid; 3-(2,2-Disphosphonoethyl)-1-methyl-1-(2-mercaptoethyl)piperidiniumchlorid; und den pharmazeutisch annehmbaren Salzen und Estern hievon besteht.

**8.** Verwendung eines hochwirksamen Phosphonats nach Anspruch 1, wobei die genannte hochwirksame Phosphonatverbindung von der Gruppe ausgewählt ist, welche aus N-(4-Hydroxy-4,4-diphosphonobutyl)-N,N,N-trimethylammoniumiodid; N-(3-Hydroxy-3,3-diphosphonopropyl)-N,N-dimethyl-N-pentylammoniumiodid und den pharmazeutisch annehmbaren Salzen und Estern hievon besteht.

**9.** Verwendung eines hochwirksamen Phosphonats nach Anspruch 1, wobei die genannte hochwirksame Phosphonatverbindung von der Gruppe ausgewählt ist, welche aus [(5-[Mercaptomethyl]-2-piperidinyl)methylen]bis[phosphonsäure]; [(5-Mercaptomethyl-3-piperidinyl)methylen]bis[phosphonsäure]; [(5-Mercapto-2-piperidinyl)methylen]bis[phosphonsäure]; [(5-[4-Mercaptobutyl]-2-piperidinyl)methylen]bis[phosphonsäure]; [(5-Mercapto-3-piperidinyl)methylen]bis[phosphonsäure]; [(5-[5-Mercaptopentyl]-3-piperidinyl)methylen]bis[phosphonsäure]; [(5-[2-Mercaptoethyl]-4-piperidinyl)methylen]bis[phosphonsäure]; [(5-Mercapto-4-piperidinyl)methylen)bis[phosphonsäure]; [2-(5-Mercapto-2-piperidinyl)ethyliden]bis[phosphonsäure]; [2-(5-[3-Mercaptopropyl]-2-piperidinyl)ethyliden]bis[phosphonsäure]; [2-(5-Mercapto-3-piperidinyl)ethyliden]bis[phosphonsäure]; [2-(5-Mercapto-4-piperidinyl)ethyliden]bis[phosphonsäure]; [2-(5-[4-Mercaptobutyl]-2-piperidinyl)ethyliden]bis[phosphonsäure]; [2-(5-Mercaptomethyl-3-piperidinyl)ethyliden]bis[phosphonsäure]; [(2-[5-Mercapto-2-piperidinyl]-1-hydroxy)ethyliden]bis[phosphonsäure]; [(2-[5-(3-Mercaptopropyl)-2-piperidinyl]-1-hydroxy)ethyliden]bis[phosphonsäure]; [(2-[5-Mercapto-3-piperidinyl]-1-hydroxy]ethyliden]bis[phosphonsäure]; [(2-[5-[2-Mercaptoethyl)-3-piperidinyl]-1-hydroxy)ethyliden]bis[phosphonsäure]; [(2-[5-Mercapto-4-piperidinyl]-1-hydroxy)ethyliden]bis[phosphonsäure]; [(2-[5-Mercaptomethyl-4-piperidinyl]-1-hydroxy)ethyliden]bis[phosphonsäure]; [(2-[5-Mercaptomethyl-3-methyl-2-piperidinyl]-1-hydroxy)ethyliden]bis[phosphonsäure]; [(2-[5-Mercapto-3-methyl-2-piperidinyl]-1-hydroxy)ethyliden]bis[phosphonsäure]; [(2-[3-Mercaptomethyl-5-methyl-2-piperidinyl]-1-hydroxy)ethyliden]bis[phosphonsäure]; [2-(5-Mercaptomethyl-3-methyl-2-piperidinyl)ethyliden]bis[phosphonsäure]; [2-(3-Mercaptomethyl-5-methyl-2-piperidinyl)ethyliden]bis[phosphonsäure]; [3-[5-(Mercaptomethyl)-2-piperidinyl]propyliden]bis[phosphonsäure]; [3-[5-(Mercaptomethyl)-3-piperidinyl]propyliden]bis[phosphonsäure]; [3-[5-(Mercaptomethyl)-4-piperidinyl]propyliden]bis[phosphonsäure]; [3-[5-(Mercaptomethyl)-2-piperidinyl]-1-hydroxypropyliden]bis[phosphonsäure]; [3-[5-(Mercapto-3-piperidinyl]-1-hydroxypropyliden]bis[phosphonsäure]; [3-[5-(4-Mercaptobutyl)-4-piperidinyl]-1-hydroxyropyliden]bis[phosphonsäure]; [2-(3-(Mercaptomethyl-5-methyl-2-pyridinyl)ethyliden]bis[phosphonsäure]; [2-(5-[3-(Mercaptopropyl]-2-methyl-2-piperidinyl)ethyliden]bis[phosphonsäure]; [(2-[5-(2-Mercaptopropyl]-2-piperidinyl]-1-amino)ethyliden]bis[phosphonsäure]; [(2-[5-(3-Mercaptopropyl]-3-piperidinyl]-1-amino)ethyliden]bis[phosphonsäure]; 2-(5-[3-Mercaptopropyl]-4-piperidinyl)-1-aminoethyliden]bis[phosphonsäure]; [(2-[3-Methyl-5-(3-mercaptopropyl)-2-piperidinyl]-1-hydroxy)ethyliden]bis[phosphonsäure]; [(2-[3-Amino-5-(3-mercaptopropyl)-2-piperidinyl]-1-hydroxy)ethyliden]bis[phosphonsäure]; [2-[5-Mercapto-2-(1,4-diazinyl)]ethyliden]bis[phosphonsäure]; [2-[5-(3-Mercaptopropyl)-2-(1,4-diazinyl)]ethyliden]bis[phosphonsäure]; [2-[5-(3-Mercaptopropyl)-2-(1,4-diazinyl)]-1-hydroxyethyliden]bis[phosphonsäure]; [2-[5-Mercapto-2-(1,4-diazinyl)]-1-hydroxyethyliden]bis[phosphonsäure]; [2-[5-Mercapto-2-(1,3-diazinyl)]ethyliden]bis[phosphonsäure]; [2-[5-(3-Mercaptopropyl)-2-(1,3-diazinyl)]ethyliden]bis[phosphonsäure]; [2-[5-(3-Mercaptopropyl)-2-(1,3-diazinyl)]-1-hydroxyethyliden]bis[phosphonsäure]; [2-[5-Mercapto-2-(1,3-diazinyl)]-1-hydroxyethyliden]bis[phosphonsäure]; [(5-[3-Mercaptopropyl]-2-piperidinyl)aminomethylen]bis[phosphonsäure]; [(5-Mercapto-2-piperidinyl)aminomethylen]bis[phosphonsäure]; [(5-[3-Mercaptopropyl]-3-piperidinyl)aminomethylen]bis[phosphonsäure]; [(5-Mercapto-3-piperidlnyl)aminomethylen]bis[phosphonsäure]; [(5-Mercapto-4-piperidinyl)aminomethylen]bis[phosphonsäure]; [(5-[3-Mercaptopropyl]-4-piperidinyl)aminomethylen]bis[phosphonsäure]; [(5-Mercapco-3-methyl-2-piperidinyliden)aminomethylen]bis[phosphonsäure]; [(5-[3-Mercaptopropyl]-3-methyl-2-piperidinyliden)aminomethylen]bis[phosphonsäure]; [2(5-Mercapto-3-methyl-2-piperidinyliden)aminoethylen]bis[phosphonsäure]; [2-(5-[3-Mercaptopropyl]-3-methyl-2-piperidinyliden)aminomethylen]bis[phosphonsäure]; [(5-Mercapto-2-piperidinyliden)aminomethylen]bis[phosphonsäure]; [(5-[3-Mercaptopropyl]-2-piperidinyliden)aminomethylen]bis[phosphonsäure]; [2-(5-Mercapto-2-piperidinyliden)aminoethylen]bis[phosphonsäure]; [(5-(3-Mercaptopropyl]-2-piperidinyliden)aminomethylen]bis[phosphonsäure]; [(5-[3-Mercaptopropyl]-2-[1,4-diazinyliden])aminomethylen]bis[phosphonsäure]; [(5-[3-Mercaptopropyl]-2-[1,3-diazinyliden])aminomethylen]bis[phosphonsäure]; [(4-[3-Mercaptopropyl]-2-[1,3,5-triazinyliden])aminomethylen]bis[phosphonsäure]; N-(2'-(1',3'-Diazinyliden))aminomethandiphosphonsäure; und den pharmazeutisch annehmbaren Salzen und Estern hievon besteht.

10. Verwendung eines hochwirksamen Phosphonats nach Anspruch 1, wobei das genannte hochwirksame Phosphonat ein Phosphonosulfonat, ein Phosphonocarboxylat oder ein Phosphonoalkylphosphinat und ein pharmazeutisch annehmbares Salz oder ein pharmazeutisch annehmbarer Ester hievon ist.

## Revendications

1. Utilisation d'un phosphonate possédant une grande efficacité dans la préparation d'un médicament destiné à l'accroissement de la masse osseuse d'un malade humain ou d'un autre mammifère souffrant d'ostéoporose, comprenant une durée de traitement de trente (30) jours, ledit traitement comprenant l'administration d'un phosphonate possédant une grande efficacité dans laquelle

   (a) ladite administration d'un phosphonate possédant une grande efficacité comprenant l'administration systémique audit patient d'une quantité d'un phosphonate possédant une grande efficacité variant de 0,00001 mgP / kg à 0,1 mgP / kg par jour d'administration dudit phosphonate possédant une grande efficacité à condition que ladite quantité du phosphonate possédant une grande efficacité soit administrée au moins quotidiennement pendant la durée du traitement de trente (30) jours; et dans laquelle
   (b) ladite période de traitement de trente (30) jours peut être suivie d'une période de repos d'au moins un jour.

2. Utilisation d'un phosphonate possédant une grande efficacité selon la revendication 1, dans laquelle le médicament doit être utilisé avant qu'il se produise une perte notable de poids net du squelette chez ledit malade.

3. Utilisation d'un phosphonate possédant une grande efficacité selon la revendication 1, dans laquelle ledit phosphonate possédant une grande efficacité est un acide bis-phosphonique, un phosphinate de phosphonoalkyle, un phosphonosulfonate ou un phosphonocarboxylate, de préférence un acide bis-phosphonique ainsi que son sel ou ester acceptables d'un point de vue pharmaceutique.

4. Utilisation d'un phosphonate possédant une grande efficacité selon la revendication 1, dans laquelle ledit phosphonate possédant une grande efficacité est un composé de formule :

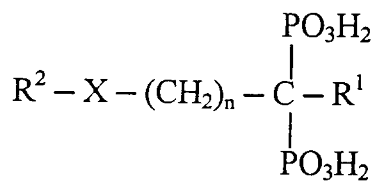

où n est un nombre entier de 0 à 7; $R^1$ représente hydrogène, chloro, amino ou hydroxy; X représente -NH-, oxygène, ou une liaison simple; $R^2$ représente un hétérocycle de 5 à sept chaînons ayant de 1 à 3 hétéroatomes, un hétérocycle de 5 à 7 chaînons contenant un azote quaternaire; ainsi que leurs sels et esters acceptables d'un point de vue pharmaceutique.

**5.** Utilisation d'un phosphonate possédant une grande efficacité selon la revendication 1, dans laquelle ledit phosphonate possédant une grande efficacité est choisi dans le groupe constitué par l'acide 4-amino-1-hydroxybutane-1,1-bis-phosphonique; l'acide 2-(3-pyridyl)-1-hydroxybutane-1,1-bis-phosphonique; l'acide 2-(*N*-imidazolyl)-1-hydroxyéthane-1,1-bis-phosphonique; l'acide 3-(*N*-pentyl-*N*-méthyl)-amino-1-hydroxypropane-1,1-bis-phosphonique; l'acide 3-(*N*-pyrrolidino)-1-hydroxypropane-1,1-bis-phosphonique; l'acide 2-(6-pyrrolopyridine)-1-hydroxyéthane-1,1-bis-phosphonique; l'acide 2-(2-pyridyl)-1-hydroxyéthane-1,1-bis-phosphonique; l'acide *N*-cycloheptylaminométhanebis-phosphonique; l'acide 3-(*N*,*N*-diméthyl)amino-1-hydroxypropane-1,1-bis-phosphonique ainsi que leurs sels et esters acceptables d'un point de vue pharmaceutique.

**6.** Utilisation d'un phosphonate possédant une grande efficacité selon la revendication 5, dans laquelle ledit phosphonate possédant une grande efficacité est le 2-(3-pyridyl)-1-hydroxyéthane-1,1-bis-phosphonique ou son sel ou ester acceptables d'un point de vue pharmaceutique.

**7.** Utilisation d'un phosphonate possédant une grande efficacité selon la revendication 1, dans laquelle ledit phosphonate possédant une grande efficacité est choisi dans le groupe constitué par l'iodure de 2-(2-hydroxy-2,2-diphosphonoéthyl)-1,1-diméthylpipéridinium; l'iodure de 3-(2-hydroxy-2,2-diphosphonoéthyl)-1-méthylpyridinium; l'hydroxyde de 3-(2-hydroxy-2,2-diphosphonoéthyl)-1-méthylpyridinium; le chlorure de 3-(2,2-diphosphonoéthyl)-1-éthylpyridinium; le chlorure de 3-(2,2-diphosphonoéthyl)-1-(2-mercaptoéthyl)pyridinium; l'hydroxyde de 2-(2-hydroxy-2,2-diphosphonoéthyl)-1-méthylpyridinium; l'hydroxyde de 3-(3-hydroxy-3,3-diphosphonopropyl)-1-méthylpyridinium; l'iodure de 3-(2,2-diphosphono-2-hydroxyéthyl)-1,1-diméthylpipéridinium; le chlorure de 3-(2,2-diphosphonoéthyl)-1-heptylpyridinium; le chlorure de 3-(2,2-diphosphonoéthyl)-1-méthylpyridinium; l'iodure de 3-(2,2-phosphonométhylphosphinoéthyl)-1-méthylpyridinium; le chlorure de 3-(2-phosphono-2-sulfonoéthyl)-1-méthylpyridinium; le chlorure de 3-(2-carboxy-2-phosphonoéthyl)-1-méthylpyridinium; le chlorure de 2-diphosphonométhyl-1,1-diméthylpipéridinium; le chlorure de 3-diphosphonométhyl-1,1-diméthylpipéridinium; le chlorure de 4-diphosphonométhyl-1,1-diméthylpipéridinium; le chlorure de 2-(2,2-diphosphonoéthyl)-1,1-diméthylpipéridinium; le chlorure de 3-(2,2-diphosphonoéthyl)-1,1-diméthylpipéridinium; le chlorure de 4-(2,2-diphosphonoéthyl)-1,1-diméthylpipéridinium; le chlorure de 2-(2,2-diphosphonoéthyl)-1-méthyl-1-(2-mercaptoéthyl)pipéridinium; le chlorure de 3-(2,2-diphosphonoéthyl)-1-méthyl-1-(2-mercaptoéthyl)pipéridinium; le chlorure de 4-(2,2-diphosphonoéthyl)-1-méthyl-1-(2-mercaptoéthyl)pipéridinium; le chlorure de 2-[2,2-diphosphono-1-(2-mercaptoéthyl)éthyl]-1,1-diméthylpipéridinium; le chlorure de 3-[2,2-diphosphono-1-(3-mercaptopropyl)éthyl]-1,1-diméthylpipéridinium; le chlorure de 4-[2,2-diphosphono-1-(2-acétylthioéthyl)éthyl]-1,1-diméthylpipéridinium; le chlorure de 2-(2,2-diphosphono-2-hydroxyéthyl)-1,1-diméthylpipéridinium; le chlorure de 3-(2,2-diphosphono-2-hydroxyéthyl)-1,1-diméthylpipéridinium; le chlorure de 4-(2,2-diphosphono-2-hydroxyéthyl)-1,1-diméthylpipéridinium; le chlorure de 2-(2,2-diphosphono-2-hydroxyéthyl)-1,1,3-triméthylpipéridinium; le chlorure de 2-(2,2-diphosphono-2-hydroxyéthyl)-1,1,5-triméthylpipéridinium; le chlorure de 2-(2,2-diphosphonoéthyl)-1,1,3-triméthylpipéridinium; le chlorure de 2-(2,2-diphosphonoéthyl)-1,1,5-triméthylpipéridinium; le chlorure de 2-(3,3-diphosphonopropyl)-1,1-diméthylpipéridinium; le chlorure de 3-(3,3-diphosphonopropyl)-1,1-diméthylpipéridinium; le chlorure de 4-(3,3-diphosphonopropyl)-1,1-diméthylpipéridinium; le chlorure de 2-(3,3-diphosphono-3-hydroxypropyl)-1,1-diméthylpipéridinium; le chlorure de 3-(3,3-diphosphono-3-hydroxypropyl) 1,1-diméthylpipéridinium; le chlorure de 4-(3,3-diphosphono-3-hydroxypropyl)-1,1-diméthylpipéridinium; le chlorure de 2-(2,2-diphosphonopropyl)-1,1-diméthylpipéridinium; le chlorure de 3-(2,2-diphosphonopropyl)-1,1-diméthylpipéridinium; le chlorure de 4-(2,2-diphosphonopropyl)-1,1-diméthylpipéridinium; le chlorure de 2-(2,2-diphosphono-2-aminoéthyl)-1,1-diméthylpipéridinium; le chlorure de 3-(2,2-diphosphono-2-aminoéthyl)-1,1-diméthylpipéridinium; le chlorure de 4-(2,2-diphosphono-2-aminoéthyl)-1,1-diméthylpipéridinium; le chlorure de 2-(2,2-diphosphono-2-aminoéthyl)-1,1,3-triméthylpipéridinium; le chlorure de 3-(2,2-diphosphono-2-aminoéthyl)-1,1,5-triméthylpipéridinium; le chlorure de 2-(2,2-diphosphono-2-(méthylamino)éthyl)-1,1-diméthylpipéridinium; le chlorure de 2-(4,4-diphosphono-4-hydroxybutyl)-1,1,3-triméthylpipéridinium; le chlorure de 2-(4,4-diphosphono-4-hydroxybutyl)-1,1-diméthylpipéridinium; le chlorure de 2-(2,2-diphosphono-2-hydroxyéthyl)-3-carboxy-1,1-diméthylpipéridinium; le chlorure de 2-(2,2-diphosphono-2-hydroxyéthyl)-5-carboxy-1,1-diméthylpipéridinium, le chlorure de 2-(2,2-diphosphonoéthyl)-1-méthylpyrimidinium; le chlorure de 4-(2,2-diphosphonoéthyl)-1-méthylpyrimidinium; le chlorure de 2-(2,2-diphosphono-2-hydroxyéthyl)-1-méthylpyrimidinium; le chlorure de 4-(2,2-diphosphono-2-hydroxyéthyl)-1-méthylpyrimidinium; le chlorure de 2-(3,3-diphosphonopropyl)-1-méthylpyrimidinium; le chlorure de 4-(3,3-diphosphonopropyl)-1-méthylpyrimidinium; le chlorure de 2-(3,3-diphosphono 1-hydroxypropyl)-1-méthylpyrimidinium; le chlorure de 4-(3,3-diphosphono-1-hydroxypropyl)-1-méthylpyrimidinium; le chlorure de 2-(2,2-diphosphono-2-aminoéthyl)-1-méthylpyrimidinium; le chlorure de 3-[(diphosphonométhyl)oxo]-1,1-diméthylpipéridinium; le chlorure de 4-[(diphosphonométhyl)oxo]-1,1-diméthylpipéridinium; le chlorure de 3-[(2,2-diphosphonoéthyl)oxo]-1,1-diméthylpipéridinium; le chlorure de 4-[(2,2-diphosphonoéthyl)oxo]-1,1-diméthylpipéridinium; le chlorure de 3-[(diphosphonométhyl)thio]-1,1-diméthylpipéridinium; le chlorure de 4-[(diphosphonométhyl)thio]-1,1-diméthylpipéridinium; l'iodure de 3-(2-hydroxy-2,2-diphosphonoéthyl)-1-méthylpyridinium; l'hydroxyde de 3-(2-hydroxy-2,2-diphosphonoéthyl)-1-méthylpyridinium; le chlorure de 3-(2,2-diphosphonoéthyl)-1-(2-mercaptoéthyl)pyridinium; l'iodure de 2-(2-hydroxy-2,2-diphosphonoéthyl)-1,1-diméthylpipéridinium; l'iodure de 3-(2,2-diphosphono-2-hydroxyéthyl)-1,1-diméthylpipéridinium; le chlorure de 3-(2,2-diphosphonoéthyl)-1-heptylpyridinium; le chlorure de 3-(2,2-diphosphonoéthyl)-1-méthylpyridinium; le chlorure de 2-(2,2-diphosphonoéthyl)-1,1-diméthylpipéridinium; le chlorure de 3-(2,2-diphosphonoéthyl)-1,1-diméthylpipéridinium; le chlorure de 4-(2,2-diphosphonoéthyl)-1,1-diméthylpipéridinium; le chlorure de 2-(2,2-diphosphono-2-hydroxyéthyl)-1,1-diméthylpipéridinium; le chlorure de 3-(2,2-diphosphono-2-hydroxyéthyl)-1,1-diméthylpipéridinium; le chlorure de 4-(2,2-diphosphono-2-hydroxyéthyl)-1,1-diméthylpipéridinium; le chlorure de 2-(2,2-diphosphono-2-hydroxyéthyl)-1,1,3-triméthylpipéridinium; le chlorure de 2-(2,2-diphosphono-2-hydroxyéthyl)-1,1,5-triméthylpipéridinium; le chlorure de 2-[2,2-diphosphono-1-(2-mercaptoéthyl)éthyl]-1,1-diméthylpipéridinium; le chlorure de 3-[2,2-diphosphono-1-(3-mercaptopropyl)éthyl]-1,1-diméthylpipéridinium; le chlorure de 2-(2,2-diphosphonoéthyl)-1-méthyl-1-(2-mercaptoéthyl)pipéridinium; le chlorure de 3-(2,2-diphosphonoéthyl)-1-méthyl-1-(2-mercaptoéthyl)pipéridinium; le chlorure de 4-(2,2-diphosphonoéthyl)-1-méthyl-1-(2-mercaptoéthyl)pipéridinium; l'iodure de 3-(2-hydroxy-2,2-diphosphonoéthyl)-1-méthylpyridinium; l'hydroxyde de 3-(2-hydroxy-2,2-diphosphonoéthyl)-1-méthylpyridinium; le chlorure de 3-(2,2-diphosphonoéthyl)-1-(2-mercaptoéthyl)pyridinium; le chlorure de 2-[2,2-diphosphono-1-(2-mercaptoéthyl)éthyl]-1,1-diméthylpipéridinium; le chlorure de 3-[2,2-diphosphono-1-(3-mercaptopropyl)éthyl]-1,1-diméthylpipéridinium; le chlorure de 2-(2,2-diphosphonoéthyl)-1-méthyl-1-(2-mercaptoéthyl) pipéridinium; le chlorure de 3-(2,2-diphosphonoéthyl)-1-méthyl-1-(2-mercaptoéthyl)pipéridinium; et leurs sels et esters acceptables d'un point de vue pharmaceutique.

8. Utilisation d'un phosphonate possédant une grande efficacité selon la revendication 1, dans laquelle ledit phosphonate possédant une grande efficacité est choisi dans le groupe constitué par l'iodure de *N*-(4-hydroxy-4,4-diphosphonobutyl)-*N,N,N*-triméthylammonium; l'iodure de *N*-(3-hydroxy-3,3-diphosphonopropyl)-*N,N*-diméthyl-*N*-pentylammonium et leurs sels et esters acceptables d'un point de vue pharmaceutique.

9. Utilisation d'un phosphonate possédant une grande efficacité selon la revendication 1, dans laquelle ledit phosphonate possédant une grande efficacité est choisi dans le groupe constitué par l'acide [5-(mercaptométhyl)pipéridin-2-ylméthylène]bis-phosphonique; l'acide [5-(mercaptométhyl)pipéridin-3-ylméthylène]bis-phosphonique; l'acide [5-mercaptopipéridin-2-ylméthylène]bis-phosphonique; l'acide [5-(4-mercaptobutyl)pipéridin-2-ylméthylène]bis-phosphonique; l'acide [5-mercaptopipéridin-3-ylméthylène]bis-phosphonique; l'acide [5-(5-mercaptopentyl) pipéridin-3-ylméthylène]bis-phosphonique; l'acide [5-(2-mercaptoéthyl) pipéridin-4-ylméthylène]bis-phosphonique; l'acide [(5-mercaptopipéridin-4-yl)méthylène]bis-phosphonique; l'acide [2-(5-mercaptopipéridin-2-yl)éthylidène]bis-phosphonique; l'acide [2-(5-[3-mercaptopropyl]pipéridin-2-yl)éthylidène]bis-phosphonique; l'acide [2-(5-mercaptopipéridin-3-yl) éthylidène]bis-phosphonique; l'acide [2-(5-mercaptopipéridin-4-yl) éthylidène]bis-phosphonique; l'acide [2-(5-[4-mercaptobutyl]pipéridin-2-yl)éthylidène]bis-phosphonique; l'acide [2-(5-mercaptométhyl)pipéridin-3-yl)éthylidène]bis-phosphonique; l'acide [2-(5-mercaptopipéridin-2-yl)-1-hydroxyéthylidène]bis-phosphonique; l'acide [(2-[5-(3-mercaptopropyl)pipéridin-2-yl]-1-hydroxyéthylidène]bis-phosphonique; l'acide [(2-[5-mercaptopipéridin-3-yl]-1-hydroxy)éthylidène]bis-phosphonique; l'acide [(2-[5-(2-mercaptoéthyl)pipéridin-3-yl]-1-hydroxy)éthylidène]bis-phosphonique; l'acide [(2-[5-mercaptopipéridin-4-yl]-1-hydroxy)éthylidène]bis-phosphonique; l'acide [(2-[5-mercaptométhylpipéridin-4-yl]-1-hydroxy)éthylidène]bis-phosphonique; l'acide [(2-[5-mercaptométhyl-3-méthylylpipéridin-2-yl]-1-hydroxy)éthylidène]bis-phosphonique; l'acide [2-(5-mercapto-3-méthyl)pipéridin-2-yl]-1-hydroxy)éthylidène]bis-phosphonique; l'acide [(2-[3-mercaptométhyl-5-méthylpipéridin-2-yl]-1-hydroxy)éthylidène]bis-phosphonique; l'acide [2-(5-mercaptométhyl-3-méthylpipéridin-2-yl)éthylidène]bis-phosphonique; l'acide

[2-(3-mercaptométhyl-5-méthylpipéridin-2-yl)éthylidène]bis-phosphonique; l'acide [3-(5-mercaptométhylpipéridin-2-yl)propylidène]bis-phosphonique; l'acide [3-(5-mercaptométhylpipéridin-3-yl)propylidène]bis-phosphonique; l'acide [3-(5-mercaptornéthylpipéridin-4-yl)propylidène]bis-phosphonique; l'acide [3-(5-mercaptométhylpipéridin-2-yl)-1-hydroxypropylidène]bis-phosphonique; l'acide [3-(5-mercaptopipéridin-3-yl)-1-hydroxypropylidène]bis-phosphonique; l'acide [3-[5-(4-mercaptobutyl)pipéridin-4-yl]-1-hydroxypropylidène]bis-phosphonique; l'acide [2-(3-(mercaptométhyl-5-méthylpyridin-2-yl)éthylidène]bis-phosphonique; l'acide [2-(5-[3-mercaptopropyl]-2-méthylpipéridin-2-yl)éthylidène]bis-phosphonique; l'acide [(2-[5-[2-mercaptopropyl]pipéridin-2-yl]-1-amino)éthylidène]bis-phosphonique; l'acide [(2-[5-[3-mercaptopropyl]pipéridin-3-yl]-1-amino)éthylidène]bis-phosphonique; l'acide [(2-[5-(3-mercaptopropyl)pipéridin-4-yl]-1-amino)éthylidène]bis-phosphonique; l'acide [(2-[3-méthyl-5-(3-mercaptopropyl)pipéridin-2-yl]-1-hydroxy)éthylidène]bis phosphonique; l'acide [(2-[3-amino-5-(3-mercaptopropyl)pipéridin-2-yl]-1-hydroxy)éthylidène]bis-phosphonique; l'acide [2-[5-mercapto-2-(1,4-diazinyl)]-1-hydroxyéthylidène] bis-phosphonique; l'acide [2-[5-(3-mercaptopropyl)-2-(1,4-diazinyl)]-1-hydroxyéthylidène]bis-phosphonique; l'acide [2-[5-mercapto-2-(1,4-diazinyl)]-1-hydroxyéthylidène]bis-phosphonique; l'acide [2-[5-mercapto-2-(1,3-diazinyl)]-éthylidène]bis-phosphonique; l'acide [2-[5-(3-mercaptopropyl)-2-(1,3-diazinyl)]éthylidène]bis-phosphonique; l'acide [2-[5-(3-mercaptopropyl)-2-(1,3-diazinyl)]-1-hydroxyéthylidène]bis-phosphonique; l'acide [2-[5-mercapto-2-(1,3-diazinyl)]-1-hydroxyéthylidène]bis-phosphonique; l'acide [(5-[3-mercaptopropyl]pipéridin-2-yl)aminométhylène]bis-phosphonique; l'acide [(5-mercaptopipéridin-2-yl)aminonméthylène]bis-phosphonique; l'acide [(5-[3-mercaptopropyl]pipéridin-3-yl)aminométhylène]bis-phosphonique; l'acide [(5-mercaptopropyl]pipéridin-3-yl)aminométhylène]-bis-phosphonique; l'acide [(5-mercaptopipéridin-3-yl)aminométhylène]bis-phosphonique; l'acide [(5-mercaptopipéridin-4-yl)aminontéthylène]bis-phosphonique; l'acide [(5-[3-mercaptopropyl]pipéridin-4-yl)aminométhylène]bis-phosphonique; l'acide [(5-mercapto-3-méthyl]pipéridin-2-ylidène)aminométhylène]bis-phosphonique; l'acide [(5-[3-mercaptopropyl]-3-méthylpipéridin-2-ylidène)aminométhylène]bis-phosphonique; l'acide [2-(5-mercapto-3-méthylpipéridin-2-ylidène) aminoéthylène]bis-phosphonique; l'acide [2-(5-[3-mercaptopropyl]-3-méthylpipéridin-2-ylidène)aminonméthylène]bis-phosphonique; l'acide [(5-mercaptopipéridin-2-ylidène)aminométhylène]bis-phosphonique; l'acide [(5-[3-mercaptopropyl]pipéridin-2-ylidène)aminométhylène]bis-phosphonique; l'acide [2-(5-mercaptopipéridin-2-ylidène)aminoéthylènebis-phosphonique; l'acide [(5-[3-mercaptopropyl]pipéridin-2-ylidène]aminométhylènebis-phosphonique; l'acide [(5-[3-mercaptopropyl]-2-[1,4-diazinylidène])aminométhylène]bis-phosphonique; l'acide [(5-[3-mercaptopropyl]-2-[1,3-diazinylidène])aminométhylène]bis-phosphonique; l'acide [(4-[3-mercaptopropyl]-2-[1,3,5-triazinylidène])aminométhylène]bis-phosphonique; l'acide *N*-[2'-(1',3'-diazinylidène)]-aminométhane diphosphonique; et leurs sels et esters acceptables d'un point de vue pharmaceutique.

**10.** Utilisation d'un phosphonate possédant une grande efficacité selon la revendication 1, dans laquelle ledit phosphonate possédant une grande efficacité est un phosphonosulfonate, un phosphonocarboxylate ou un phosphonoalkylphosphinate et son sel ou ester acceptables d'un point de vue pharmaceutique.